# EUROPEAN PATENT SPECIFICATION

(11) **EP 4 677 620 B1**
(45) Date of publication and mention of the grant of the patent: **04.11.2026**
(21) Application number: 24707778.7
(22) Date of filing: 28.02.2024
(51) Int. Cl.: G16H 50/20, G01N 21/00

(54) **METHODS AND DEVICES FOR PROVIDING BLACKLIST INFORMATION ON THE SUITABILITY OF A MOBILE DEVICE FOR PERFORMING ANALYTICAL MEASUREMENTS**
VERFAHREN UND VORRICHTUNGEN ZUR BEREITSTELLUNG VON BLACKLIST-INFORMATIONEN ÜBER DIE EIGNUNG EINER MOBILEN VORRICHTUNG ZUR DURCHFÜHRUNG ANALYTISCHER MESSUNGEN
PROCÉDÉS ET DISPOSITIFS POUR FOURNIR DES INFORMATIONS DE LISTE NOIRE CONCERNANT LA PERTINENCE D'UN DISPOSITIF MOBILE POUR EFFECTUER DES MESURES ANALYTIQUES

(30) Priority: 03.03.2023 EP 23159993
(43) Date of publication of application: 14.01.2026
(73) Proprietor: F. Hoffmann-La Roche AG, 4070 Basel (CH); Roche Diabetes Care GmbH, 68305 Mannheim (DE)
(72) Inventor: LIMBURG, Bernhard, 68305 Mannheim (DE); BERG, Max, 68305 Mannheim (DE); HAILER, Fredrik, 68305 Mannheim (DE)
(74) Representative: Riwotzki, Karsten
(86) International application number: PCT/EP2024/055020
(87) International publication number: WO 2024/184138

(56) References cited:
- US-A1- 2020 242 769
- US-A1- 2020 350 069

## Description

### Technical Field

The present invention relates to computer-implemented methods of operating a cloud server for providing blacklist information on the suitability of mobile devices for performing analytical measurements, of operating a mobile device, specifically for the purpose of using the mobile device for an analytical measurement, and of operating a system comprising at least one cloud server and a plurality of mobile devices. The invention further relates to an analytical method for determining at least one parameter of a sample of the bodily fluid. The invention further relates to corresponding computer programs and computer-readable storage media, as well to a corresponding cloud server, a corresponding mobile device and a corresponding analytical system. The invention specifically may be used in medical diagnostics in order to quantitatively or qualitatively detect one or more properties of a sample of a bodily fluid. As an example, the methods and devices may be used for qualitatively and/or quantitatively detecting the presence of one or more analytes in a bodily fluid, such as for detecting glucose in one or more of blood, urine, saliva and/or interstitial fluid. Additionally or alternatively, as an example, the invention may be used for detecting the presence of the SARS-CoV-2 coronavirus in a sample of a bodily fluid. Other fields of application of the present invention, however, are also feasible.

### Background art

In the field of medical diagnostics, in many cases, one or more analytes have to be detected in samples of a body fluid, such as blood, interstitial fluid, urine, saliva or other types of body fluids. Examples of analytes to be detected are viruses, such as the SARS-CoV-2 coronavirus. However, the present invention may also be used for other types of analytes, such as glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, in the field of glucose detection, with regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

As a further example, for point-of-care (POC) testing or for other situations where a quick result is desirable, a variety of lateral flow (LF) tests detecting viral antigens are commercially available. For example, in the case of SARS-CoV-2, LF test detecting the nucleocapsid antigen are widely used, but also the spike antigen may be detected. However, also tests detecting viral nucleic acids have been proposed as lateral flow tests, cf. e.g. WO 2021/228839 and references cited therein, which are also referred to as "SHERLOCK" tests. Further, instead of blood tests, saliva was proposed as a suitable sample material for SARS-CoV-2 detection (Wyllie et al. (2020), medRxiv 2020.04.16.20067835; doi.org/10.1101/2020.04.16.20067835). B. Ince and M. K. Sezgintürk provide in "Lateral flow assays for viruses diagnosis: Up-to-date technology and future prospects", 2022, TrAC Trends in Analytical Chemistry, Volume 157, 116725, an overview of current problems and accessible solutions in detecting infectious agents and diseases by lateral flow assay (LFA), focusing on increasing sensitivity with various detection methods. Further, the sensitivity of SARS-CoV-2 antigen rapid diagnostic tests (Ag RDT) was evaluated in "Comparative sensitivity evaluation for 122 CE-marked rapid diagnostic tests for SARS-CoV-2 antigen, Germany, September 2020 to April 2021", H. Scheiblauer et al., Eurosurveillance, 26, 2100441 (2021).

In analytical measurements, specifically analytical measurements based on optical detection such as color formation reactions, one technical challenge resides in the evaluation of the color change which is due to the detection reaction. Besides using dedicated analytical devices, such as stationary or handheld blood glucose meters, the use of generally available electronics such as smart phones and portable computers or other mobile devices has become more and more popular over the recent years. Thus, a camera comprised by these mobile devices may be used to measure the color change of the detection reaction. Generally, products on the market typically make use of color reference, such as color reference cards, for photometric measurements, for example to compensate for different lighting conditions during the measurement. One or more images of a color reference having color reference fields with known reference color values may be captured by using the camera of the mobile device. A transformation algorithm may be determined by relating the measured reference color values to the known reference color values.

A technical challenge, when using mobile devices such as smart phones for analytical measurements, resides in the fact that a wide variety of mobile devices exists on the market, with a plurality of pre-known or unknown operating systems. When using a mobile device for analytical purposes, the suitability of the mobile device for the specific analytical purpose, thus, typically has to be evaluated. In addition, the specific hardware and/or software properties of the mobile device have to be taken into account, e.g. in order to compensate for differing image processing within the mobile device.

EP 3 477 270 A1 discloses a method for evaluating the suitability of a mobile device having at least one camera for the purpose of performing an analytical measurement based on a color formation reaction. The method comprises: a) providing the at least one mobile device having the at least one camera; b) providing at least one object having at least one reference color field; c) taking at least one image of at least part of the reference color field by using the camera; and d) deriving at least one item of color resolution information by using the image. Similarly, EP 3 477 286 A1 discloses a method for evaluating the suitability of a mobile device for performing an analytical measurement. The mobile device has at least one camera. The method comprises: a) providing the at least one mobile device having the at least one camera; b) providing at least one reference object having at least one predefined spatial extension in at least one spatial dimension; c) taking at least one image of at least part of the reference object by using the camera; and d) deriving at least one item of spatial resolution information by using the image.

WO 2014/011488 A2 and EP 2870555 B1 disclose a machine-executed method of continuous analyte monitoring, comprising transmitting, using a computing device, an identification of a version of an operating system of the computing device and/or a continuous analyte monitoring (CAM) software application installed on the computing device to a remote server, the CAM software application comprising instructions, when executed by a processor of the computing device, that cause the computing device to process and display analyte concentration information. The method further comprises receiving a response from the remote server responsive indicative of a compatibility check of the version of the operating system with the version of the CAM software application; and producing an output to responsive to the response.

US 11456076 B2 describes validation to ensure the compatibility and correct performance of medical devices critical to patient health and safety communicating with third party hardware and software, such as smart devices. Disclosed are methods and systems to perform a self-validation of hardware and/or software components with a medical device to confirm that any combination of hardware and software are compatible and perform acceptably. If the self-validation passes then the user may safely use the system, and it may report to a cloud server that the particular configuration tested was successful. If the self-validation fails, the user will be prevented from using safety-critical aspects of the application and be notified of the incompatibility; and the results may also be reported to a cloud server.

US 11424027 B2 discloses a method for adjusting the operation of a surgical instrument using machine learning in a surgical suite. Inter alia, a list of surgical tools and/or modular components may be declared unavailable/unusable/unauthorized to communicate with and/or access a surgical hub and/or cloud-based analytics system.

WO 2014/011488 A2 and EP 2 870 555 B1 disclose systems and methods for leveraging smartphone features in continuous glucose monitoring. Specifically, a machine-executed method for checking compatibility of the operating system of a computing device that is a smart phone or tablet computer using a commercially available operating system and a continuous analyte monitoring, CAM, software application installed thereon is disclosed. Therein, a remote server is used for providing a compatibility check indicating if the version of the operating system is compatible with the CAM software application.

US 2020/242769 A1 discloses a method for evaluating the suitability of a mobile device having a camera for performing an analytical measurement. The method includes providing the mobile device and providing a reference object having a predefined spatial extension in a spatial dimension. An image is taken of at least part of the reference object using the camera and is used to derive spatial resolution information that includes one or more numerical values that quantify the capability of the camera to resolve two or more objects in the image. A threshold value based on a minimum number of pixels in the spatial dimension within a region of interest in the image is provided and the spatial resolution information is compared with the threshold value. It is determined from the comparison whether the mobile device is suitable for performing the analytical measurement.

US 2010/010406 A1 discloses medical devices critical to patient health and safety that communicate with third party hardware and software, such as smart devices, require validation to ensure compatibility and correct performance. Disclosed are methods and systems to perform a self-validation of hardware and/or software components with a medical device to confirm that any combination of hardware and software are compatible and perform acceptably. If the self-validation passes then the user may safely use the system, and it may report to a cloud server that the particular configuration tested was successful. If the self-validation fails, the user will be prevented from using safety-critical aspects of the application and be notified of the incompatibility; and the results may also be reported to a cloud server.

Despite the advantages achieved by the known methods and devices, several technical challenges remain. Thus, specifically, known methods and systems generally relate to the compatibility of the mobile devices on a macroscopic scale, specifically to software compatibility of the mobile devices' software and operating system with the intended medical purpose. Other systems generally relate to anti-counterfeiting and the prevention of using unauthorized hardware for specific medical purposes. These systems and methods, however, are widely unsuited to take into account individual mobile devices' properties, both hardware related and software related, which may have a significant impact on the result of the medical purpose to be performed with the mobile devices, specifically the analytical measurement to be performed with the help of the mobile device. Thus, specifically in the field of detecting optical detection reactions, e.g. by using one or more test elements such as color formation test strips, individual properties of the mobile devices such as color resolution and/or spatial resolution may have a significant impact on the analytical result. There is, thus, a need for further improving user-friendliness and failsafe functions of systems using mobile devices for analytical measurements.

### Problem to be solved

It is therefore desirable to provide devices and methods which at least partially address the above-mentioned challenges. Specifically, it is desirable to provide devices and methods which allow for user-friendly analytical measurements by using mobile devices having at least one camera, with a low failure rate, taking into account the mobile devices' individual properties.

### Summary

This problem is addressed by computer-implemented methods of operating a cloud server for providing blacklist information on the suitability of mobile devices for performing analytical measurements, of operating a mobile device, and of operating a system comprising at least one cloud server and a plurality of mobile devices with the features of the independent claims. This problem is further addressed by an analytical method for determining at least one parameter of a sample of the bodily fluid, by corresponding computer programs and computer-readable storage media, as well by a corresponding cloud server, a corresponding mobile device and a corresponding analytical system with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention, which is defined by the appended claims, and without any restriction regarding the possibility of combining the features introduced in such way with other optional or non-optional features of the invention.

In a first aspect, a computer-implemented method of operating a cloud server for providing blacklist information is disclosed. The computer-implemented method of operating a cloud server for providing blacklist information comprises the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The term "computer implemented" as used herein is a broad term and is to be given its ordinary and customary meaning to a person of ordinary skill in the art and is not to be limited to a special or customized meaning. The term specifically may refer, without limitation, to a method which is performed by using computer programming, and/or by using at least one computer and/or at least one computer network. Thus, as an example, one or more or even all of the method steps may be performed by appropriate software, e.g. by using computer-readable instructions which, when executed on a computer or a computer network, cause the computer or computer network to perform the method steps. The term "software" as used herein may, specifically, refer to a computer program. The computer and/or computer network may comprise at least one processor which is configured for performing at least one, more than one or all of the method steps of the method according to the present invention. Specifically, each of the method steps is performed by the computer and/or computer network. The method may be performed completely automatically, specifically without user interaction.

The method comprises the following steps:
i. providing a cloud-based blacklist database on the cloud server, the blacklist database comprising blacklist information on a plurality of mobile devices, each mobile device having at least one camera, the blacklist information comprising information on the suitability of at least one of the mobile devices' hardware and the mobile devices' software for performing at least one analytical measurement, specifically at least one analytical measurement based on an optical detection reaction, specifically based on a color formation reaction, more specifically an optical detection reaction using an optical test element;
ii. providing, upon request by a requesting mobile device of the plurality of mobile devices, at least one item of blacklist information from the blacklist database to the requesting mobile device, the blacklist information comprising information on the suitability of at least one of the requesting mobile device's hardware and the requesting mobile device's software for performing the at least one analytical measurement, wherein the request from the requesting mobile device comprises at least one item of information on the mobile device comprising an individual identifier of the mobile device and the model type of the mobile device;
iii. receiving, specifically by the cloud server, at least one item of individual check result information from a sending mobile device of the plurality of mobile devices, the item of check result information comprising information on the suitability of at least one of the sending mobile device's hardware and the sending mobile device's software for performing the at least one analytical measurement; and
iv. evaluating, specifically by the cloud server, the item of individual check result information for the necessity of updating the blacklist information on the blacklist database.

In a step i., a cloud-based blacklist database on the cloud server is provided, the blacklist database comprising blacklist information on a plurality of mobile devices, each mobile device having at least one camera, the blacklist information comprising information on the suitability of at least one of the mobile devices' hardware and the mobile devices' software for performing at least one analytical measurement, specifically at least one analytical measurement based on an optical detection reaction, specifically based on a color formation reaction, more specifically an optical detection reaction using an optical test element.

The term "server" as used herein is a broad term and is to be given its ordinary and customary meaning to a person of ordinary skill in the art and is not to be limited to a special or customized meaning. The term specifically may refer, without limitation, to a device which is capable of performing one or more operations with the data to be processed. Specifically, the server may comprise at least one processor, which may be programmed, by appropriate software, for performing one or more operations with the data to be processed. The term "cloud server" may indicate that the server is a cloud-based server established by at least one computer cloud or a plurality of computers, specifically a remote device, such as a separate device that receives data from and/or transfers data to the mobile device, particularly via a computer network, such as the internet, more particularly by using at least one respective connection interface. The cloud server may be accessed by the mobile device from a plurality of different mobile device locations. As an example, the connection interface may be configured for providing access of the cloud server and/or of the mobile device to the computer network, specifically in a manner that data may be received and/or transmitted by the respective device via the computer network. The connection interface may be or may comprise at least one port comprising one or more of a network or internet port.

The term "database" as used herein is a broad term and is to be given its ordinary and customary meaning to a person of ordinary skill in the art and is not to be limited to a special or customized meaning. The term specifically may refer, without limitation, to an organized collection of data, generally stored and accessed electronically, e.g. on at least one volatile and/or at least one non-volatile data storage device, specifically by a computer or computer system. Thus, the database may comprise the organized collection of data itself or the at least one storage device having stored thereon the organized collection of data. In this context, the term "providing" may relate to the fact that the organized collection of data is made available, e.g. by itself, e.g. by providing access to the data, and/or to the process of storing the data in an organized form on the at least one data storage device.

The database, as outlined above, may comprise or may be comprised by at least one data storage device. The database, in addition to the organized collection of data itself, may comprise one or more additional software components and/or one or more additional items of data, such as one or more software components for organizing the data and/or software components for making the data available, such as search functions. Thus, the database may comprise at least one data base management system comprising a software running on a computer or computer system, such as on the cloud server, the software allowing for interaction with one or more of a user, an application or the database itself, such as in order to capture and analyze the data contained in the database. The database management system may further encompass facilities to administer the database. The database, containing the data, may, thus, be comprised by a database system which, besides the data, comprises one or more associated items of information and/or one or more associated applications. The database may specifically be a cloud-based blacklist database. The cloud-based blacklist database may have the data storage device independent from the mobile device and may be accessible via at least one data connection, such as a computer network, specifically a wireless data connection. The cloud-based blacklist database may be stored on the cloud server and/or accessible via the cloud server.

The term "blacklist" as used herein is a broad term and is to be given its ordinary and customary meaning to a person of ordinary skill in the art and is not to be limited to a special or customized meaning. The term specifically may refer, without limitation, to an arbitrary list comprising information on a plurality of technical entities to be subject to at least one technical limitation, particularly when considering the technical functionality of the entity. The technical entity may be a mobile device. The technical limitation may be related to the permission of performing of the at least one analytical measurement by using the mobile device. The technical functionality may be or may comprise the performing of at least one analytical measurement. Thus, the at least one technical limitation specifically may relate to the suitability or non-suitability of at least one of the mobile devices' hardware and the mobile devices' software for performing at least one analytical measurement. The blacklist, specifically, may list technical entities which are found to be at least one of limited, unsuited and impermissible for performing the technical functionality. Additionally or alternatively, the blacklist may list technical entities which are found at least one of not to be limited, to be suited and to be permissible for performing the technical functionality. Again, additionally or alternatively, the blacklist may list information on technical entities, the information generally relating to at least one of the limitation or non-limitation, the suitability or non-suitability and the permissibility or non-permissibility of the respective technical entity for performing the technical functionality.

The term "suitability" as used herein is a broad term and is to be given its ordinary and customary meaning to a person of ordinary skill in the art and is not to be limited to a special or customized meaning. The term specifically may refer, without limitation, to a property of an element or device fulfilling at least one condition indicating the capability of the element or device of performing one or more predetermined functions. Thus, as an example, the suitability may be qualified or quantified by using one or more characteristic parameter(s) of the device. These one or more characteristic parameters may, individually or according to a predetermined combination, be compared with one or more conditions. As a simple example, the individual parameters or one or more of the parameters may be compared with one or more comparative values, reference values or standard values, wherein the comparison may be a qualitative or a quantitative comparison and may result in a binary result such as "suited" or "not suited"/"unsuited". As an example, the at least one comparative or reference value may comprise at least one threshold value. Additionally or alternatively, however, the comparison may result in a quantitative result, such as a figure indicating a degree of suitability. The comparative values, reference values or standard values may be derived, as an example, from experiments or from boundary conditions determined e.g. by the precision to be achieved.

The term "information on the suitability" as used herein is a broad term and is to be given its ordinary and customary meaning to a person of ordinary skill in the art and is not to be limited to a special or customized meaning. The term specifically may refer, without limitation, to an indication or information regarding the suitability, specifically in the present case of the suitability of the mobile device for the purpose of performing the analytical measurement based on the optical detection reaction. The item of suitability information, as an example, may be Boolean or digital information, such as indicating "suited" or "not suited"/"unsuited". Alternatively, however, the suitability may also be quantified.

The term "mobile device" as used herein is a broad term and is to be given its ordinary and customary meaning to a person of ordinary skill in the art and is not to be limited to a special or customized meaning. The term specifically may refer, without limitation, to a mobile electronics device, specifically a personal mobile device (PDA), more specifically to a mobile communication device such as a cell phone and/or a smartphone. Additionally or alternatively, the mobile device may also refer to a notebook, a tablet computer or another type of portable computer, such as a wearable, specifically smart glasses, having at least one camera. Alternatively or in addition, a smartphone having an external camera may be used. The external camera may be comprised by spectacles, particularly smart glasses. The mobile device may have a direct internet access, particularly in a manner that the mobile device is free of being required to connect to a further network, such as a Wireless Local Area Network, WLAN, to connect to the internet. Thus, generally, the mobile devices may be selected from the group consisting of: a cell phone having at least one camera, specifically a smart phone; a portable computer having at least one camera, specifically at least one of a notebook and a tablet computer. The mobile device may be used as a field device, particularly for performing analytical measurements.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using the camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications, such as notebook computers, tablets or, specifically, cell phones such as smartphones. Thus, specifically, the camera may be part of the mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. A larger number of color values is also feasible, such as four colors for each pixel, for example R, G, G, B. Color cameras are generally known to the skilled person. Thus, as an example, each pixel of the camera chip may have three or more different color sensors, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B or C, M, Y, K or the like. The color sensitivities of the pixels may be generated by color filters or by appropriate intrinsic sensitivities of the sensor elements used in the camera pixels. These techniques are generally known to the skilled person.

The determining of the analyte concentration may comprise an optical detection. As used herein, the term "optical detection" refers to a detection of a reaction using an optical test chemical, such as a color-change test chemical, which changes in color in the presence of the analyte. The color change specifically may depend on the amount of analyte present in the sample. Techniques for determining the analyte by optical detection and in particular analyzing color of the spot on the test filed are generally known to the skilled person. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction.

The term "optical test element" as used herein is a broad term and is to be given its ordinary and customary meaning to a person of ordinary skill in the art and is not to be limited to a special or customized meaning. The term specifically may refer, without limitation, to an arbitrary element or device configured for performing an optical detection reaction, for example a color-change detection reaction and/or a reaction during which one or more optically detectable features on or within the test element become visible, such as one or more markings, such as linear markings known from rapid COVID testing. The optical test element may, as an example, be embodied as a test stick or as a test element. The optical test element may particularly have the at least one reagent test region, such as a "test field", containing at least one test chemical being sensitive for the property of the sample, such as for detecting the at least one analyte. The optical test element may, as an example, comprise one or more application sites for applying the at least one sample. The application site may be different from the position of the at least one reagent test region and may be fluidically connected to the reagent test region, such as by one or more capillary elements, such as one or more porous elements capable of transporting liquid. The optical test element, as an example, may comprise at least one substrate, such as at least one carrier, with the at least one reagent test region applied thereto or integrated therein. The optical test element may comprise at least one control area, specifically in a proximity to the reagent test region, for example enclosing or surrounding the reagent test region and/or arranged behind the reagent test region in a direction of flow of the sample of bodily fluid on the optical test element. The control area may be a separate field independently arranged on the substrate or carrier. The control area may be configured for indicating correct applying of the sample of the bodily fluid on the optical test element. The carrier, as an example, may be strip-shaped, thereby rendering the optical test element a test strip. These test strips are generally widely in use and available. One test strip may carry a single reagent rest region or a plurality of reagent test regions having identical or different test chemicals comprised therein. Additionally or alternatively, the optical test element may be embodied as a stick or chip, e.g. with a housing having the above-mentioned substrate disposed therein, e.g. a housing having one or more application openings for applying the sample and one or more detection windows for enabling an optical detection of the at least one detection reaction. Various options are feasible.

The optical test element may be a digital-type test element for digitally detecting the presence or absence of at least one predetermined analyte in the sample. Thus, the optical test element may be a test element capable of providing the information "positive" if the at least one predetermined analyte is determined to be present in the sample, or "negative" if the at least one predetermined analyte is determined not to be present in the sample. The optical test element may be configured for changing at least one optically detectable property of at least one feature when the analyte is detected in the sample, whereas the at least one optically detectable property of the at least one feature may be kept unchanged when the analyte is not detected in the sample. As an example, the optical test element may be a SARS-CoV-2 rapid antigen test, specifically a SARS-CoV-2 & Flu A/B rapid antigen test.

The term "reagent test region" as used herein is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the reagent test region having the test chemical comprised therein. Other layers may be present providing specific optical properties such as reflective properties, providing spreading properties for spreading the sample or providing separation properties such as for separating of particulate components of the sample, such as cellular components. The sample of the bodily fluid may be applied directly to the reagent test region, e.g. for blood glucose measurements where a droplet of blood may be directly applied to the test strip comprising the reagent test region, or may be applied indirectly to the reagent test region, e.g. by applying the sample of the bodily fluid to a reservoir or an application site of the optical test element, wherein the sample of the bodily fluid may flow from the reservoir of application site to the reagent test region of the optical test element, such as by capillary forces acting on the sample of the bodily fluid.

In a step ii., upon request by a requesting mobile device of the plurality of mobile devices, at least one item of blacklist information is provided from the blacklist database to the requesting mobile device, the blacklist information comprising information on the suitability of at least one of the requesting mobile device's hardware and the requesting mobile device's software for performing the at least one analytical measurement.

The term "requesting", or any grammatical variation thereof, as used herein is a broad term and is to be given its ordinary and customary meaning to a person of ordinary skill in the art and is not to be limited to a special or customized meaning. The term specifically may refer, without limitation, to a first unit querying an item, such as data, from a second unit. The requesting specifically may take place electronically, e.g. by sending an electronic request, e.g. wirelessly, such as via at least one telecommunications network. The requested data , specifically may comprise at least one the item of information, such as the blacklist information. As a consequence of a request, the requested information may be provided from the second unit to the first unit, e.g. wirelessly, such as via the same telecommunications network as used for the request itself and/or via a different telecommunications network. Requesting may comprise sending requesting information to the second unit. Requesting may comprise sending identification information, particularly associated with the requesting entity, such as the first unit. Before transmitting the requested data to the first unit, the second unit may consider the provided identification information, particularly to identify the requested data and/or authenticate the first unit. The identification may comprise evaluating the identification information.

The term "providing", or any grammatical variation thereof, as used herein is a broad term and is to be given its ordinary and customary meaning to a person of ordinary skill in the art and is not to be limited to a special or customized meaning. The term specifically may refer, without limitation, to making the provided item available, e.g. to the public or to a specific device and/or entity. In the example discussed above, the providing specifically may take place by transmitting data from a first unit to a second unit and/or vice versa. The data may comprise at least one item of information, such as the blacklist information.

In a step iii., at least one item of individual check result information is received, specifically by the cloud server, from a sending mobile device of the plurality of mobile devices, the item of check result information comprising information on the suitability of at least one of the sending mobile device's hardware and the sending mobile device's software for performing the at least one analytical measurement. For receiving the individual check result information, the individual check result information is generated by the mobile device and transmitted from the mobile device to the cloud server, particularly by a computer network and/or by using connection interfaces.

The term "item of check result information" as used herein is a broad term and is to be given its ordinary and customary meaning to a person of ordinary skill in the art and is not to be limited to a special or customized meaning. The term specifically may refer, without limitation, to an item of information generated in a test, wherein the item of information is associated to the outcome of the test. The item of check result information may be derived in anon-board check of the mobile device. The result and/or outcome may directly or indirectly indicate a suitability, specifically in the present case the suitability of the mobile device for the purpose of performing the analytical measurement based on the optical detection reaction. as discussed above, the item of check result information specifically may comprise information in a Boolean format, such as "suited" or unsuited and/or may comprise other types of information, such as specific information on the type of test and the outcome, in a Boolean format and/or a different format, such as by providing test results.

In a step iv., the item of individual check result information is evaluated, specifically by the cloud server, for the necessity of updating the blacklist information on the blacklist database. The blacklist database may be updated based on the evaluated necessity, specifically automatically, more specifically by the cloud server and/or by a further device. The updated blacklist information may be available in subsequent mobile device requests.

The term "updating", or any grammatical variation thereof, as used herein is a broad term and is to be given its ordinary and customary meaning to a person of ordinary skill in the art and is not to be limited to a special or customized meaning. The term specifically may refer, without limitation, to bringing something to the latest, current state, present condition, or current point in time. Updating may comprise or be an actualization of a computer program and/or a database. For evaluating the necessity of updating the blacklist information, the blacklist information may be checked to be in line and/or agree with the received individual check result information. In case, the blacklist information is in line and/or agree with the individual check result information an update of the blacklist information may not be necessary. In case, the blacklist information is not in line and/or does not agree with the individual check result information an update of the blacklist information may be necessary. Particularly for updating the cloud-based blacklist database, the blacklist information may be amended in a manner that the comprised information is in line and/or agrees with the latest received item of check result information.

The mobile devices of the plurality of mobile devices are grouped into model groups, wherein the blacklist information comprises model-related blacklist information and individual blacklist information, wherein the model-related blacklist information comprises, for the individual model groups, information on the suitability of at least one of the hardware and the software of the mobile devices belonging to the respective model group for performing the at least one analytical measurement, and wherein the individual blacklist information comprises individual information on the suitability of at least one of the hardware and the software of individual mobile devices of the plurality of mobile devices for performing the at least one analytical measurement.

The term "model" as used herein is a broad term and is to be given its ordinary and customary meaning to a person of ordinary skill in the art and is not to be limited to a special or customized meaning. The term specifically may refer, without limitation, to a type of an entity characterized by at least one certain characteristic feature and/or property. The assignment to a specific model may take place automatically, and/or by a manufacturer or provider of the mobile device. Thus, the manufacturer and/or provider may assign the mobile device and/or the software running on the mobile device to at least one specific models, e.g. by assigning a model number and/or a software number. The at least one certain characteristic feature may be at least one hardware component of the mobile device and/or a software running on the mobile device. The term "model group" as used herein is a broad term and is to be given its ordinary and customary meaning to a person of ordinary skill in the art and is not to be limited to a special or customized meaning. The term specifically may refer, without limitation, to a plurality of entities having the at least one certain characteristic feature in common. The at least one characteristic feature may be association with the suitability of at least one of the mobile devices' hardware and the mobile devices' software for performing the analytic measurement. The hardware component may be associated with at least one item of individual check result information derived when evaluating the suitability of at least one of the mobile devices' hardware and the mobile devices' software. Alternatively or in addition, the at least one characteristic may be associated to a software, particularly a software having a selected functionality, associated with at least one item of individual check result information derived when evaluating the suitability of at least one of the mobile devices' hardware and the mobile devices' software. Such a software may be an operating system and/or a driver of the camera of the mobile device. An individual mobile device may be comprised by one model group and/or a plurality of model groups.

**In** the blacklist information, model groups denoted to be unsuited for performing the at least one analytical measurement may be blacklisted. The term "blacklisting" as used herein is a broad term and is to be given its ordinary and customary meaning to a person of ordinary skill in the art and is not to be limited to a special or customized meaning. The term specifically may refer, without limitation, to put an entity on a blacklist, particularly the cloud-based blacklist database. By blacklisting a model group, any mobile device being comprised by a model group may be denoted as being unsuited for performing the at least one analytical measurement. **In** the blacklist information, individual mobile devices denoted to be unsuited for performing the at least one analytical measurement may be blacklisted. Thereby, the individual mobile device may be denoted as being unsuited for performing the at least one analytical measurement. The blacklisting of an individual mobile device may be independent of a blacklisting of a model group being associated with the individual mobile device.

The method may comprise determining whether the suitability for performing an analyte measurement of an individual mobile phone and/or a model group may be related to a specific software running on the respective mobile device. Particularly in case the suitability for performing an analyte measurement of an individual mobile phone and/or a model group is related to a specific software running on the respective mobile device, the method further may comprise monitoring software updates of the mobile devices. For monitoring the software updates of an individual mobile device, a software version may be monitored. In case a reference to an individual mobile device in the blacklist database comprises an individual identifier of the mobile device, particularly an International Mobile Equipment Identity, IMEI, number of the mobile device, and a software version, an updated mobile device may be considered as a new mobile device in the database, particularly in a manner that a new entry in the database is generated by using a new reference considering the updated software version. The method further may comprise updating the blacklist information on the blacklist database in case the software was updated. Monitoring software updates may comprise, particularly frequently, requesting information on current version of the respective software from a database running on the mobile device, such as a database comprised by the mobile device. Particularly, updates of the operating systems of the mobile devices may be monitored.

In case a blacklist information, particularly a model related blacklist information, is associated with a particular version of a software, a blacklisted model group and/or individual mobile device may be considered suitable for performing an analyte measurement once the software is updated. Particularly in such a case, the blacklist database may be updated in a manner that the model group and/or individual mobile device may be indicated as not being blacklisted in the blacklist database.

Once the blacklist database may be updated in a manner that the model group and/or individual mobile device may be indicated as not blacklisted in the blacklist database, a threshold condition may be updated to an updated threshold condition, particularly an update threshold condition different from the threshold condition, particularly an updated threshold condition having a decreased demand for blacklisting the blacklisted model group and/or individual mobile device again. Exemplarily, a model type subj ected to the updated threshold condition may be blacklisted after only one failure of a mobile in an on-board suitability check.

The method may comprise recording at least a part of the individual check result information from sending mobile devices having sent individual check result information to the cloud server. For recording the part of the individual check result information, part of the individual check result information may be stored in a database associated with the cloud server.

The recording may comprise updating at least one check result counter with each sending of individual check result information by a sending mobile device of the plurality of mobile devices. The check result counter may comprise at least one of a model check result counter for a model group of the sending mobile device and an individual check result counter for the individual sending mobile device.

The term "check result counter" as used herein is a broad term and is to be given its ordinary and customary meaning to a person of ordinary skill in the art and is not to be limited to a special or customized meaning. The term specifically may refer, without limitation, to an item of information configured for comprising information on the number of received check results. The item of information may be a numerical value or an equivalent thereof. The item of information may comprise information on a number of failed on-board suitability checks indicating that the individual sending mobile device is unsuited for performing the analytical measurement. Alternatively or in addition, the item of information may comprise information on a number of passed on-board suitability checks indicating that the individual sending mobile device is suited for performing the analytical measurement. The method may make use of one dedicated check result counter for each mobile device and/or of one or more check result counters for groups of mobile devices, such as groups of mobile devices, of the same model group.

Step iv. may comprise subjecting the recorded part of the individual check result information for the individual sending mobile devices to at least one threshold condition.

The threshold condition may comprise at least one of the following threshold conditions:
- comparing an individual absolute number of failures of the individual sending mobile device's on-board suitability check, each failure indicating that the individual sending mobile device it is unsuited for performing the analytical measurement, with a predetermined individual absolute failure threshold, e.g. by checking if the individual absolute number of failures is above the individual absolute failure threshold;
- comparing a model absolute number of failures with a predetermined model absolute failure threshold, wherein the model absolute number of failures comprises a total number of failures of individual sending mobile devices' on-board suitability checks of mobile devices belonging to a specific model group, each failure indicating that the sending mobile device is unsuited for performing the analytical measurement, e.g. by checking if the absolute number of failures is above the predetermined model absolute failure threshold;
- comparing an individual relative number of failures, the individual relative number of failures indicating a ratio of failed on-board suitability checks and passed on-board suitability checks of the individual sending mobile device's on-board suitability checks with a predetermined individual relative failure threshold, e.g. by checking if the individual relative number of failures is above the predetermined individual relative failure threshold;
- comparing a model relative number of failures, the model relative number of failures indicating a ratio of failed on-board suitability checks and passed on-board suitability checks of individual sending mobile devices' on-board suitability checks of sending mobile devices belonging to a specific model group, with a predetermined model relative failure threshold, e.g. by checking if the model relative number of failures is above the predetermined model relative failure threshold.

If the subjecting of the recorded part of the individual check result information for the individual sending mobile devices to the at least one threshold condition may indicate that the individual sending mobile device is unsuited for performing the analytical measurement, the individual sending mobile device may be blacklisted in the blacklist information.

If the subjecting of the recorded part of the individual check result information for the individual sending mobile devices to at least one threshold condition may indicate that a specific model group to which the sending mobile device belongs is unsuited for performing the analytical measurement, the specific model group is blacklisted in the blacklist information.

**In** a further aspect, the present invention relates to a computer-implemented method of operating a mobile device having at least one camera comprising the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The method comprises the following steps:
a. the mobile device requesting, from at least one cloud server, at least one item of blacklist information, the blacklist information comprising information on the suitability of at least one of the mobile device's hardware and the mobile device's software for performing at least one analytical measurement based on an optical detection reaction, wherein the request from the requesting mobile device comprises at least one item of information on the mobile device comprising an individual identifier of the mobile device and the model type of the mobile device;
b. the mobile device evaluating the item of blacklist information;
c. if the result of the evaluation in step b. indicates that the mobile device is unsuited for the analytical measurement, the mobile device preventing the analytical measurement;
d. if the result of the evaluation in step b. indicates that the mobile device is suited for the analytical measurement, the mobile device initiating at least one on-board suitability check for on-board checking the suitability of the mobile device for performing the analytical measurement;
e. if the result of the on-board suitability check in step d. indicates that the mobile device is unsuited for the analytical measurement, the mobile device preventing the analytical measurement and sending individual check result information to the cloud server, the individual check result information comprising update information on the failed on-board suitability check of the mobile device;
f. if the result of the onboard suitability check in step d. indicates that the mobile device is suited for the analytical measurement, the mobile device providing clearance information for the analytical measurement.

For details, options and definitions, reference may be made to any aspect, embodiment or claim discussed elsewhere herein. The mobile device may comprise at least one camera and/or at least one processor.

The term "on-board check" as used herein is a broad term and is to be given its ordinary and customary meaning to a person of ordinary skill in the art and is not to be limited to a special or customized meaning. The term specifically may refer, without limitation, to a self-diagnostic and reporting test of an entity, specifically the mobile device, particularly for testing the suitability of the mobile device for performing the analytical measurement. The on-board check specifically may comprise using the mobile device's own resources. Thus, the on-board check specifically may be performed at least partially automatically. In addition, however, one or more check accessories may be used for the on-board check, e.g. by using one or more reference cards, as will be outlined in further detail below. Thus, in addition to the automatic checking routine as mentioned above, one or more manual check steps may be comprised by the on-board check. The steps of the on-board check, specifically all of the steps of the on-board check, may be performed at the location of the respective mobile device, e.g. by the mobile device itself and/or by a user handling the mobile device.

The term "the mobile device evaluating the item of blacklist information" as used herein is a broad term and is to be given its ordinary and customary meaning to a person of ordinary skill in the art and is not to be limited to a special or customized meaning. The term specifically may refer, without limitation, to the mobile device considering the item of blacklist information to process a suitability of the mobile device for the analytical measurement, particularly to decide whether step c. or step d. of the method are to be processed. As indicated in the above, the blacklist information may comprise information on the suitability of at least one of the mobile device's hardware and the mobile device's software for performing the at least one analytical measurement based on an optical detection reaction. As further indicated in the above, the item of suitability information refers to an indication or information regarding the suitability, specifically in the present case of the suitability of the mobile device for the purpose of performing the analytical measurement based on the optical detection reaction. As an example, the item of suitability information may be Boolean or digital information, such as indicating "suited" or "not suited"/"unsuited".

Alternatively or in addition, the item of suitability information, may be at least one of
- an individual absolute number of failures;
- a model absolute number of failures;
- an individual relative number of failures;
- model relative number of failures.

For a further definition of these numbers, reference is made to the definitions given above for the respective number for the cloud server. At least one of these numbers, specifically the individual absolute number of failures, may be stored on the mobile device, particularly on a storage device of the mobile device. For determining the suitability of the mobile device for the analytical measurement, the mobile device, alternatively or in addition to the cloud server, may subject the respective number to at least one threshold condition. For a further definition of the threshold condition and/or the procedure on deriving the ability of the mobile device for the analytical measurement by considering the threshold condition, reference is made to respective detail discussed for the cloud server.

The computer-implemented method of operating a mobile device may further comprise:
g. recording at least one image of at least one part of at least one test field of at least one optical test element; and
h. determining at least one parameter of a sample of a bodily fluid applied to the optical test element by evaluating the image.

The term "recording at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "recording at least one image" may comprise recording a single image and/or a plurality of images such as a sequence of images. For example, the recording of the image may comprise recording continuously a sequence of images such as a video or a movie. The recording of the at least one image may be initiated by a user action or may automatically be initiated, e.g. once the presence of the at least one object within a field of view and/or within a predetermined sector of the field of view of the camera is automatically detected. These automatic image acquisition techniques are known e.g. in the field of automatic bar-code readers, such as from automatic barcode reading apps. The recording of the images may take place, as an example, by acquiring a stream or "live stream" of images with the camera, wherein one or more of the images, automatically or by user interaction such as pushing a button, are stored and used as the at least one first image or the at least one second image, respectively. The image acquisition may be supported by the processor of the mobile device.

The term "sample of a bodily fluid" as used herein is a broad term and is to be given its ordinary and customary meaning to a person of ordinary skill in the art and is not to be limited to a special or customized meaning. The term specifically may refer, without limitation, to an arbitrary aliquot part or aliquant part of a biological fluid which directly is a bodily fluid or which is derived from a bodily fluid, such as by one or more pre-processing steps, e.g. by transferring a bodily fluid to at least one sampling fluid, by diluting a bodily fluid, by centrifugation a bodily fluid or the like. The bodily fluid may comprise one or more of saliva, blood, interstitial fluid, urine or other types of body fluids. The sample of the bodily fluid may be collected via at least one nasopharyngeal swab, at least one swab of the anterior nares or from saliva, such by applying a cotton swab to a surface of the anterior nares and/or the throat. The collected sample of bodily fluid may be transferred to at least sampling or reagent fluid by immersing the cotton swab in the sampling or reagent fluid. The sampling or reagent fluid may specifically comprise lysis reagents. Alternatively or additionally, the sample of the bodily fluid may be a droplet of a body fluid as gathered from the body of a person, such as a droplet of saliva and/or blood and/or interstitial fluid or the like. The sample of the bodily fluid may specifically comprise at least one preparation of the bodily fluid, such as a cell preparation of the bodily fluid, e.g. a stained cell preparation of the bodily fluid. The sample of bodily fluid may also be simply referred to as the sample or the test sample.

At least one failsafe operation may be applied in any one of steps g. and h., specifically at least one plausibility check, and wherein, if the plausibility check determines that a failure has occurred during any one of steps g. and h., the method further may comprise sending additional individual check result information to the cloud server, the additional individual check result information comprising update information on the failure. thus, as an example, the failsafe operation may comprise subjecting the results of one or both of steps g. and h. to at least one plausibility condition, such as by comparing the results with one or more plausibility thresholds. In case the condition is not fulfilled, the failsafe operation may provide information that the respective mobile device, at least in this specific run of the method, is unsuited for performing the at least one analytical measurement based on an optical detection reaction. The update information may be considered when updating the cloud-based blacklist database.

Step f. further may comprise sending individual check result information to the cloud server, the individual check result information comprising update information on the passed on-board suitability check of the mobile device, the passed on-board suitability check of the mobile device indicating that the mobile device is suited for performing the analytical measurement.

The computer-implemented method of operating a mobile device may further comprise, in any one of steps c. and e., generating at least one indication to the user of the mobile device indicating that the mobile devices unsuited for the analytical measurement. The indication may be a haptic, auditive and/or visual indication. A visual indication may be displayed on a display of the mobile device.

The on-board suitability check in step d. may comprise at least one of: checking if the mobile device's hardware complies with one or more predetermined hardware compliance criteria; checking if the camera's color resolution complies with one or more predetermined color resolution criteria; checking if the camera's spatial resolution complies with one or more spatial resolution criteria.

The on-board suitability check of the camera's color resolution may involve considering at least one item of color resolution information. For considering, the at least one item of color resolution information may be compared with at least one threshold value. The item of color resolution information may be derived by evaluating an image taken by mobile device. The item of color resolution information may comprise one or more numerical values, which quantify the capability of resolving two or more colors. The threshold value may be selected by considering a desired precision of the analytical measurement.

The on-board suitability check of the camera's spatial resolution may involve comparing at least one item of spatial resolution information, particularly which is derived by evaluating an image taken by mobile device, with at least one threshold value. The item of spatial resolution information may comprise one or more numerical values, which corresponds to a relationship between a distance in the image and the corresponding distance in the real world. The threshold value may be selected by considering a desired precision of the analytical measurement.

The on-board suitability check in step d. may comprise capturing at least one reference image of a reference card and comparing at least one item of information derived from the reference image with at least one predetermined reference criterion. The reference card may comprise at least one of: a plurality of color reference fields, each color reference field having predetermined color coordinates; a plurality of gray level reference fields, each gray level reference field having a predetermined gray level; a plurality of position markers, the position markers having a predetermined spatial relationship.

The term "color reference field" as used herein is a broad term and is to be given its ordinary and customary meaning to a person of ordinary skill in the art and is not to be limited to a special or customized meaning. The term specifically may refer, without limitation, to a locally distributed area over the color reference card, specifically over a part of the color reference card visible in the image. The color reference card may further comprise at least two color reference fields having different reference color values. Specifically, the color reference card may comprise a plurality of color reference fields having different color values, wherein the color values of the color reference fields may be selected from a predetermined color subspace of the color space. The predetermined color subspace may comprise at least one color value of the color formation reaction of the reagent test field. The predetermined reference criterion may be selected by considering a desired precision of the analytical measurement.

For further possible definitions and/or embodiments of the color reference card, reference may be made to one or both of EP 3 477 270 A1 and EP 3 477 286 A1, as discussed above. Other embodiments, however, are also feasible.

In step a., at least one item of information may be transmitted to the cloud server by the requesting mobile device, the item of information may be selected from the group consisting of: an individual identifier of the mobile device, particularly an International Mobile Equipment Identity, IMEI, number; a model type of the mobile device; a software version of software running on the mobile device, specifically of an operating system. The method may be initiated by a user, starting an analytical measurement request on the mobile device.

In a further aspect, the present invention relates to an analytical method for determining at least one parameter of a sample of a bodily fluid, specifically for detecting at least one analyte in the bodily fluid, comprising the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The term "analytical method" as used herein is a broad term and is to be given its ordinary and customary meaning to a person of ordinary skill in the art and is not to be limited to a special or customized meaning. The term specifically may refer, without limitation, to a quantitative and/or qualitative determination of the at least one property of the sample of the bodily fluid, such as at least one of a physical, a chemical and a biological property. The determination of the at least one property may specifically comprise quantitatively or qualitatively detecting at least one analyte in the sample of the bodily fluid. The result of the analytical method, as an example, may be at least one item of information on the at least one property of the sample, such as at least one item of information on the presence or absence of an analyte of interest. Alternatively or additionally, the result of the analytical method may comprise at least one item of information indicating a concentration of the at least one analyte of interest. The analyte may be or may comprise at least one arbitrary, dedicated and/or predetermined chemical or biological substance or species, such as at least one molecule or at least one chemical and/or biological compound. For example, the analyte may be or may comprise at least one specific virus and/or any parts thereof. The result of the analytical method may be or may comprise at least one item of information indicating the presence or absence of the virus or parts thereof in the sample of the bodily fluid. For example, the analyte may be a chemical compound which takes part in metabolism, such as one or more of glucose, lactate, cholesterol or triglycerides. In this example, the analytical method may be a blood glucose measurement and, thus, the result of the analytical method may for example be a blood glucose concentration. Additionally or alternatively, other types of analytes or parameters may be determined, such as a pH value or the like. As will be understood by the skilled person, a "presence" of an analyte, for example, may be a presence of said analyte in an amount above a detection limit of the test used.

The method comprises the following steps:
- running the computer-implemented method of operating a mobile device on the mobile device, wherein the mobile device has at least one camera,
- in case the mobile device is providing clearance information for the analytical measurement in step f, receiving an image of the optical test element by the camera of the mobile device, wherein the optical test element has at least one test field configured for performing at least one optically detectable detection reaction in the presence of at least one analyte in a sample of bodily fluid applied to the optical test element, wherein the image comprises at least a part of the test field having the sample of bodily fluid applied thereto.

For details, options and definitions, reference may be made to any aspect, embodiment or claim discussed elsewhere herein. The method may be a computer implemented method. For receiving the image of the optical test element by the camera of the mobile device, the image may be captured by the camera of the mobile device. The user may be indicated that the clearance information is provided, particularly by indicating to the user that an image of the optical test element may be captured. Thereby, an analytic measurement may be prevented and/or stopped in advance without the user having to provide a sample of bodily fluid and/or apply the sample of bodily fluid to the test field.

In a further aspect, the present invention relates to a computer-implemented method of operating a system comprising at least one cloud server and a plurality of mobile devices, each mobile device having at least one camera, the method comprising:
- operating a cloud server by using the computer-implemented method of operating a cloud server for providing blacklist information; and
- operating at least one of the mobile devices of the plurality of mobile devices by using the computer-implemented method of operating a mobile device.

For details, options and definitions, reference may be made to any aspect, embodiment or claim discussed elsewhere herein. The term "system" as used herein is a broad term and is to be given its ordinary and customary meaning to a person of ordinary skill in the art and is not to be limited to a special or customized meaning. The term specifically may refer, without limitation, to an arbitrary set of interacting or interdependent components parts forming a whole. The system may comprise multiple components, for example at least two components or even more. The at least two components may be handled independently or may be coupled or connectable. The components of the system may interact with each other in order to fulfill at least one common function. The method of operating a cloud server and the method of operating at least one of the mobile devices are interrelated in the sense that they co-operate.

In a further aspect, the present invention relates to a cloud server for providing blacklist information, the cloud server, optionally having at least one processor, being configured, specifically by software configuration, for performing the computer-implemented method of operating a cloud server. The cloud server may comprise at least one processor. For details, options and definitions, reference may be made to any aspect, embodiment or claim discussed elsewhere herein.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math co-processor or a numeric co-processor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) and/or one or more tensor processing unit (TPU) and/or one or more chip, such as a dedicated machine learning optimized chip, or the like. The processor specifically may be configured, such as by software programming, for performing one or more evaluation operations as will be outlined in further detail below.

In a further aspect, the present invention relates to a computer program comprising instructions which, when the program is executed by the cloud server according to the preceding Embodiment, cause the cloud server to perform the computer-implemented method of operating a cloud server for providing blacklist information. The computer program may be executed on at least one processor comprised by the cloud server. For details, options and definitions, reference may be made to any aspect, embodiment or claim discussed elsewhere herein

In a further aspect, the present invention relates to a computer-readable storage medium, specifically a non-transient computer-readable storage medium, comprising instructions which, when the instructions are executed by the cloud server, cause the cloud server to perform the computer-implemented method of operating a cloud server for providing blacklist information. As used herein, the term "computer-readable storage medium" specifically may refer to non-transitory data storage means, such as a hardware storage medium having stored thereon computer-executable instructions. The computer-readable storage medium specifically may be or may comprise a storage medium such as a random-access memory (RAM) and/or a read-only memory (ROM). For details, options and definitions, reference may be made to any further aspect discussed elsewhere herein

In a further aspect, the present invention relates to a mobile device having at least one camera, and, optionally, at least one processor, the mobile device being configured, specifically by software configuration, for performing the computer-implemented method of operating a mobile device. For details, options and definitions, reference may be made to any aspect, embodiment or claim discussed elsewhere herein.

In a further aspect, the present invention relates to a computer program comprising instructions which, when the program is executed by the mobile device, cause the mobile device to perform the computer-implemented method of operating a mobile device. For details, options and definitions, reference may be made to any aspect, embodiment or claim discussed elsewhere herein.

In a further aspect, the present invention relates to a computer-readable storage medium, specifically a non-transient computer-readable storage medium, comprising instructions which, when the instructions are executed by the mobile device, cause the mobile device to perform the computer-implemented method of operating a mobile device. For details, options and definitions, reference may be made to any aspect, embodiment or claim discussed elsewhere herein

In a further aspect, the present invention relates to an analytical system comprising at least one mobile device having at least one camera and at least one cloud server. The term "analytical system", may refer to a system for performing at least one analytical function, specifically for performing at least one analytical measurement, for example at least one analytical measurement as described above. The analytical system may be further comprising at least one optical test element having at least one test field configured for performing at least one optically detectable detection reaction in the presence of at least one analyte, particularly in a sample of bodily fluid applied to the optical test element.

As an example, a method of assessing the suitability of a mobile device having a camera for performing an analytical measurement based on a color formation reaction, may comprise:
a. performing a hardware and/or a software system check, specifically comprising checking the camera's color resolution capabilities and/or the camera's spatial resolution capabilities;
b. transmitting, to a cloud server, any failed system checks;
c. determine, on the cloud server, if at least N (e.g. N = 5) mobile devices of the same type have transmitted a particular failed system check;
d. if yes, then block all mobile devices of the same type which have transmitted said particular failed system check, such that no future photo app measurement can be performed by said mobile devices of the same type;
if no, then block the mobile device which has transmitted said particular failed system check, such that no future photo app measurement can be performed by said mobile device.

The terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps of any one of the methods as indicated above may be performed by using a computer and/or a computer network, preferably by using a computer program.

The methods and devices according to the present invention may provide a large number of advantages compared with known methods and devices. Thus, by using the invention, the number of unnecessary and/or unsuccessful attempts of a user to use a specific mobile device for analytical measurements may be reduced. In case a specific type of mobile device, e.g. a specific type or model of smart phone, generates a large number of unsuccessful attempts of performing an analytical measurement, specifically during the on-board check and/or the measurement itself, this specific mobile device and/or the type or model of the mobile device may be blacklisted. Thereby, future use of the model and/or the specific mobile device may be prevented. In addition, the user may be informed accordingly.

Specifically, whenever an on-board suitability check of the mobile device, e.g. the smart phone, fails and/or whenever a measurement using the mobile device, e.g. the smart phone, fails, the failure, e.g. including details of the failure such as failure states, may be reported to cloud server. In the cloud server, a check may be performed whether one or more conditions are fulfilled for updating the blacklist, e.g. if more than N (e.g. N=5) different mobile devices of the same model type report the very same failure. If this is the case the mobile device model type may be put on the blacklist. Otherwise only the specific mobile device reporting the failure may be blacklisted and, as an example, blocked from further usage. Additionally or alternatively, the user may be informed that currently the used mobile device is not capable of running the measuring application and no further measurement shall be started.

In case the user does not uninstall the measuring application, particularly although the mobile device of the user is blacklisted, she/he may be informed when there is an update of the blacklist database and/or a software running on the mobile device, specifically so that an analytical measurement may be tried again. The list of blacklisted mobile device model types may be reviewed and/or updated when a major mobile device operating system release took place that may have fixed some image processing issues. Prior to this step an assessment, whether the recorded failure(s) of the mobile device may be related to a hardware, specifically a hardware component, and/or a software may be performed, particularly by considering that hardware related failures may not be fixed by an update of the operating system.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of an analytical system comprising at least one mobile device; and
- Figure 2: shows a flow chart of an exemplary embodiment of a computer-implemented method of operating a system;
- Figure 3: shows a flow chart related to monitoring software updates of the mobile devices; and
- Figure 4: shows a flow chart of an analytical method for determining at least one parameter of a sample of a bodily fluid.

### Detailed description of the embodiments

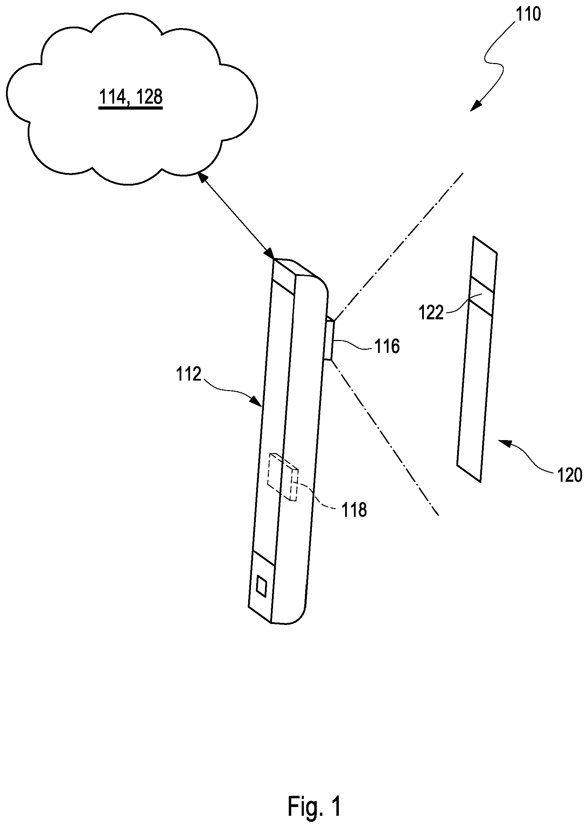

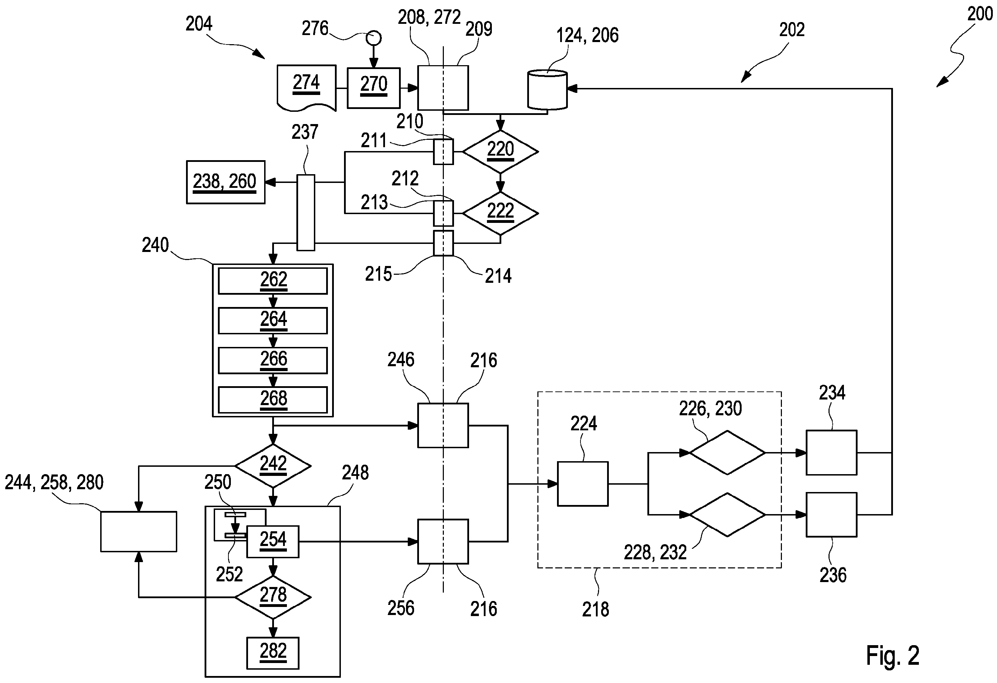

Figure 1 shows an exemplary embodiment of an analytical system 110 comprising at least one mobile device 112. The mobile device 112 comprises at least one camera 116 and at least one processor 118. The analytical system 110 may specifically comprise the mobile device 112 being configured for performing, such as by running a computer program, a method of operating the mobile device (denoted by reference number 204) according to the present invention, such as according to the exemplary embodiment shown in Figure 2 and/or according to any other embodiment disclosed herein.

The analytical system 110 further comprises at least one cloud server 114. The cloud server 114 is providing a cloud-based blacklist database 128. As shown in Figure 1, the mobile device 112 may be configured for exchanging information with the cloud-based blacklist database 128, such as by running a computer program. The information may be exchanged by transferring information to the cloud-based blacklist database 128 and/or storing information in the cloud-based blacklist database 128 and/or by retrieving information, such as requested and/or queried information, from the cloud-based blacklist database 128. The at least one cloud server 114 may be configured for performing a method of operating the cloud server 114 (denoted by reference number 202) according to the present invention, such as according to the exemplary embodiment shown in Figure 2 and/or according to any other embodiment disclosed herein.

The analytical system 110 further may comprise at least one optical test element 120 having at least one test field 122 configured for performing at least one optically detectable detection reaction in the presence of at least one analyte, particularly in a sample of bodily fluid applied to the optical test element.

Figure 2 shows a flow chart of an exemplary embodiment of a computer-implemented method of operating a system (denoted by reference number 200) comprising at least one cloud server 114 and a plurality of mobile devices 112, each mobile device 112 having at least one camera 116. The method (denoted by reference number 200) comprises: operating a cloud server 114 by using a computer-implemented method of operating a cloud server 114 for providing blacklist information (denoted by reference number 202); and operating at least one of the mobile devices 112 of the plurality of mobile devices 112 by using a computer-implemented method of operating a mobile device 112 (denoted by reference number 204). Thus, Figure 2 also shows exemplary embodiments of these methods 202 and 204.

The method of operating a cloud server 114 for providing blacklist information (denoted by reference number 201) and the method of operating a mobile device 112 (denoted by reference number 203) are separated by the dash-dotted line in Figure 2. The method of operating a cloud server 114 for providing blacklist information (denoted by reference number 202) is on the right of the dash-dotted line. The method of operating a mobile device 112 (denoted by reference number 204) is on the left of the dash-dotted line. As may be derived from Figure 2, the method of operating a cloud server 114 for providing blacklist information (denoted by reference number 202) and the method of operating a mobile device 112 (denoted by reference number 204) are interrelated in the sense that they co-operate.

The exemplary embodiment of the computer-implemented method of operating the cloud server 114 for providing blacklist information (denoted by reference number 202) comprises the following steps
i. providing a cloud-based blacklist database (denoted by reference number 206) on the cloud server 114, the blacklist database 124 comprising blacklist information on a plurality of mobile devices 112, each mobile device 112 having at least one camera 116, the blacklist information comprising information on the suitability of at least one of the mobile devices 112' hardware and the mobile devices 112' software for performing at least one analytical measurement, specifically at least one analytical measurement based on an optical detection reaction, specifically based on a color formation reaction, more specifically an optical detection reaction using an optical test element 120;
ii. providing, upon request by a requesting mobile device 112 of the plurality of mobile devices 112 (denoted by reference number 208), at least one item of blacklist information (denoted by reference number 210, 212, 214) from the blacklist database 124 to the requesting mobile device 112, the blacklist information comprising information on the suitability of at least one of the requesting mobile device's hardware and the requesting mobile device's software for performing the at least one analytical measurement, particularly the method (denoted by reference number 202) may comprise receiving a request by the requesting mobile device 112 (denoted by reference number 209);
iii. receiving, specifically by the cloud server 114, at least one item of individual check result information (denoted by reference number 216) from a sending mobile device 112 of the plurality of mobile devices 112, the item of check result information comprising information on the suitability of at least one of the sending mobile device's hardware and the sending mobile device's software for performing the at least one analytical measurement;
iv. evaluating, specifically by the cloud server 114, the item of individual check result information (denoted by reference number 218) for the necessity of updating the blacklist information on the blacklist database 124 devices.

The mobile devices 112 of the plurality of mobile devices 112 are grouped into model groups, wherein the blacklist information comprises model-related blacklist information and individual blacklist information. The model-related blacklist information comprises, for the individual model groups, information on the suitability of at least one of the hardware and the software of the mobile devices 112 belonging to the respective model group for performing the at least one analytical measurement. The individual blacklist information comprises individual information on the suitability of at least one of the hardware and the software of individual mobile devices 112 of the plurality of mobile devices 112 for performing the at least one analytical measurement. In the blacklist information, model groups denoted to be unsuited for performing the at least one analytical measurement may be blacklisted. In the blacklist information, individual mobile devices 112 denoted to be unsuited for performing the at least one analytical measurement may be blacklisted.

For providing the model-related blacklist information, particularly in step ii., the suitability of at least one of the hardware and the software of the mobile devices 112 belonging to the respective model group for performing the at least one analytical measurement associated with the requesting mobile device 112 may be considered (denoted by reference number 220). For providing the individual blacklist information, particularly in step ii., the suitability of at least one of the hardware and the software of the individual mobile device 112 for performing the at least one analytical measurement associated with the requesting mobile device 112 may be considered (denoted by reference number 222).

The method (denoted by reference number 202) may comprise recording at least a part of the individual check result information (denoted by reference number 224) from sending mobile devices 112 having sent individual check result information to the cloud server 114. The recording may comprise updating at least one check result counter with each sending of individual check result information by a sending mobile device 112 of the plurality of mobile devices 112. The check result counter comprises at least one of a model check result counter for a model group of the sending mobile device 112 and an individual check result counter for the individual sending mobile device 112.

Step iv. may comprise subjecting the recorded part of the individual check result information for the individual sending mobile devices 112 to at least one threshold condition (denoted by reference number 226, 228, 230, 232). The threshold condition may comprise at least one of the following threshold conditions:
- comparing an individual absolute number of failures of the individual sending mobile device's on-board suitability check, each failure indicating that the individual sending mobile device 112 it is unsuited for performing the analytical measurement, with a predetermined individual absolute failure threshold (denoted by reference number 226);
- comparing a model absolute number of failures with a predetermined model absolute failure threshold (denoted by reference number 228), wherein the model absolute number of failures comprises a total number of failures of individual sending mobile devices 112' on-board suitability checks of mobile devices 112 belonging to a specific model group, each failure indicating that the sending mobile device 112 is unsuited for performing the analytical measurement;
- comparing an individual relative number of failures, the individual relative number of failures indicating a ratio of failed on-board suitability checks and passed on-board suitability checks of the individual sending mobile device's on-board suitability checks with a predetermined individual relative failure threshold (denoted by reference number 230);
- comparing a model relative number of failures, the model relative number of failures indicating a ratio of failed on-board suitability checks and passed on-board suitability checks of individual sending mobile devices 112' on-board suitability checks of sending mobile devices 112 belonging to a specific model group, with a predetermined model relative failure threshold (denoted by reference number 232).

If the subjecting of the recorded part of the individual check result information for the individual sending mobile devices 112 to the at least one threshold condition indicates that the individual sending mobile device 112 is unsuited for performing the analytical measurement, the individual sending mobile device 112 is blacklisted in the blacklist information (denoted by reference number 234). Particularly therefore, the blacklist database 124 may be updated, particularly by updating the individual blacklist information.

If the subjecting of the recorded part of the individual check result information for the individual sending mobile devices 112 to at least one threshold condition indicates that a specific model group to which the sending mobile device 112 belongs is unsuited for performing the analytical measurement, the specific model group is blacklisted in the blacklist information (denoted by reference number 236). Particularly therefore, the blacklist database 124 may be updated, particularly by updating the model-related blacklist information.

The exemplary embodiment of the computer-implemented method of operating a mobile device (denoted by reference number 204) having at least one camera 116 comprises the following steps:
a. the mobile device 112 requesting, from at least one cloud server 114, at least one item of blacklist information (denoted by reference number 208), the blacklist information comprising information on the suitability of at least one of the mobile device's hardware and the mobile device's software for performing at least one analytical measurement based on an optical detection reaction, specifically based on a color formation reaction, more specifically an optical detection reaction using an optical test element 120, particularly the item of blacklist information may be received by the mobile device 112 from the providing blacklist database 124 (denoted by reference number 211, 213, 214);
b. the mobile device 112 evaluating the item of blacklist information (denoted by reference number 237);
c. if the result of the evaluation in step b. indicates that the mobile device 112 is unsuited for the analytical measurement, the mobile device 112 preventing the analytical measurement (denoted by reference number 238);
d. if the result of the evaluation in step b. indicates that the mobile device 112 is suited for the analytical measurement, the mobile device 112 initiating at least one on-board suitability check (denoted by reference number 240) for on-board checking the suitability of the mobile device 112 for performing the analytical measurement, particularly the method (denoted by reference number 204) may comprise the mobile device 112 evaluating the result of the on-board suitability check (denoted by reference number 242);
e. if the result of the on-board suitability check in step d. indicates that the mobile device 112 is unsuited for the analytical measurement, the mobile device 112 preventing the analytical measurement (denoted by reference number 244) and sending individual check result information (denoted by reference number 246) to the cloud server 114, the individual check result information comprising update information on the failed on-board suitability check of the mobile device 112;
f. if the result of the onboard suitability check in step d. indicates that the mobile device 112 is suited for the analytical measurement, the mobile device 112 providing clearance information for the analytical measurement (denoted by reference number 248).

Particularly in case clearance information for the analytical measurement is provided (denoted by reference number 248), the method may comprise performing the analytical measurement. Particularly in case clearance information for the analytical measurement is provided (denoted by reference number 248), the computer-implemented method of operating a mobile device 112 may further comprise the following steps:
g. recording at least one image (denoted by reference number 250) of at least one part of at least one test field 122 of at least one optical test element 120; and
h. determining at least one parameter (denoted by reference number 252) of a sample of a bodily fluid applied to the optical test element 120 by evaluating the image.

At least one failsafe operation (denoted by reference number 254) may be applied in any one of steps g. and h., specifically at least one plausibility check. The result of the failsafe operation may be evaluated (denoted by reference number 278). If the failsafe operation, specifically the plausibility check, determines that a failure has occurred during any one of steps g. and h., the method further may comprise sending additional individual check result information to the cloud server 114 (denoted by reference number 256), the additional individual check result information comprising update information on the failure. Additionally or alternatively, if the failsafe operation, specifically the plausibility check, determines that a failure has occurred during any one of steps g. and h., the method further may comprise preventing and/or aborting the analytical measurement (denoted by reference number 280).

Additionally or alternatively, if the failsafe operation, specifically the plausibility check, determines that no failure has occurred during any one of steps g. and h., the method further may comprise sending additional individual check result information to the cloud server 114 (denoted by reference number 256). Additionally or alternatively, if the failsafe operation, specifically the plausibility check, determines that no failure has occurred during any one of steps g. and h., the method further may comprise displaying the result of the analytical measurement (denoted by reference number 282).

The method (denoted by reference number 204) may be initiated by a user (denoted by reference number 276), starting an analytical measurement request on the mobile device 112.

In step a., the mobile device may be generating at least one item of information on the mobile device (denoted by reference number 270). Further in step a., the at least one item of information may be transmitted to the cloud server 114 (denoted by reference number 272) by the requesting mobile device 112, the item of information being selected (denoted by reference number 274) from the group consisting of: an individual identifier of the mobile device 112, particularly an International Mobile Equipment Identity, IMEI, number; a model type of the mobile device 112; a software version of software running on the mobile device 112, specifically of an operating system.

The at least one item of information on the mobile device 112 may, particularly necessarily, comprise the individual identifier of the mobile device 112, particularly the International Mobile Equipment Identity, IMEI, number, and/or the model type of the mobile device 112. The at least one item of information may additionally comprise the software version of software running on the mobile device 112. Solely providing the software version of software running on the mobile device 112 may not be sufficient.

The item of information may, specifically, comprise
- the individual identifier of the mobile device 112;
- the individual identifier of the mobile device 112 and the software version of software running on the mobile device 112;
- the model type of the mobile device 112;
- the model type of the mobile device 112 and the software version of software running on the mobile device 112;
- the individual identifier of the mobile device 112 and the model type of the mobile device 112; or
- the individual identifier of the mobile device 112, the model type of the mobile device 112 and the software version of software running on the mobile device 112.

The method (denoted by reference number 204) may further comprise, in any one of steps c. and e., generating at least one indication (denoted by reference number 258, 260), particularly to the user of the mobile device 112, indicating that the mobile device 112 is unsuited for the analytical measurement.

The on-board suitability check in step d. may comprise at least one of: checking if the mobile device's hardware complies with one or more predetermined hardware compliance criteria (denoted by reference number 262); checking if the camera's color resolution complies with one or more predetermined color resolution criteria (denoted by reference number 264); checking if the camera's spatial resolution complies with one or more spatial resolution criteria (denoted by reference number 266).

The on-board suitability check in step d. may comprise capturing at least one reference image of a reference card and comparing at least one item of information derived from the reference image with at least one predetermined reference criterion (denoted by reference number 266). The reference card may comprise at least one of: a plurality of color reference fields, each color reference field having predetermined color coordinates; a plurality of gray level reference fields, each gray level reference field having a predetermined gray level; a plurality of position markers, the position markers having a predetermined spatial relationship.

Step f. may further comprise sending individual check result information to the cloud server 114 (denoted by reference number 246), the individual check result information comprising update information on the passed on-board suitability check of the mobile device 112, the passed on-board suitability check of the mobile device 112 indicating that the mobile device 112 is suited for performing the analytical measurement.

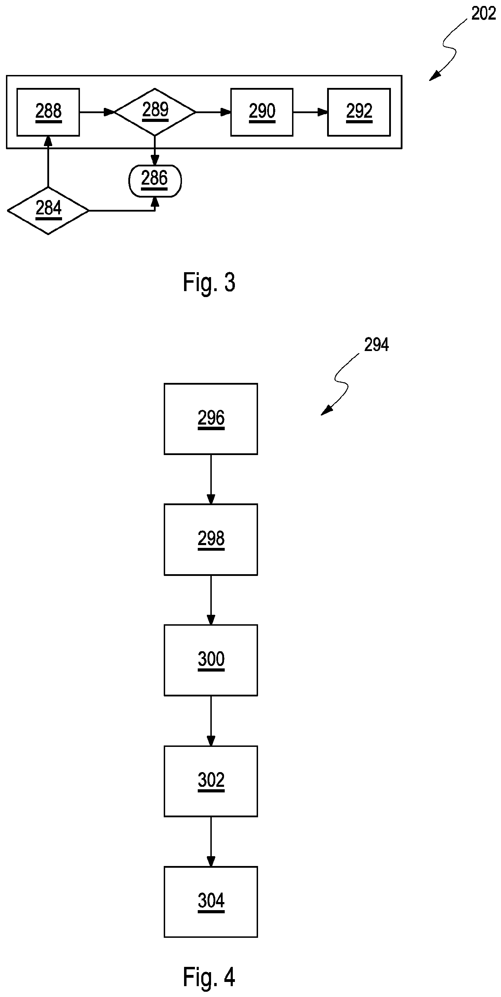

As depicted in an exemplary embodiment of Figure 3, the exemplary embodiment of the method of operating the cloud server (denoted by reference number 202) for providing blacklist information may further comprise determining whether the suitability for performing an analyte measurement of an individual mobile phone and/or a model group may be related to a specific software running on the respective mobile device(s) 112 (denoted by reference number 284). The specific software may be the operating system. Particularly in case the suitability for performing an analyte measurement of an individual mobile phone and/or a model group may not be related to a specific software running on the respective mobile device(s) 112, the method (denoted by reference number 202) may comprise maintaining the blacklist information on the individual mobile device and/or the model group, particularly in a manner that the individual mobile device and/or the model group is maintained blacklisted (denoted by reference number 286).

Particularly in case the suitability for performing an analyte measurement of an individual mobile device 112 and/or a model group may be related to a specific software running on the respective mobile device(s) 112, the method (denoted by reference number 202) may comprise monitoring software updates of the mobile device(s) 112 (denoted by reference number 288), particularly of the individual mobile device 112 and/or of the mobile devices 112 related to the model group, more particularly software updates of the specific software.

The method (denoted by reference number 202) further may comprise evaluating if a software update of the mobile devices is released (denoted by reference number 289).

Particularly in case a software update is released (denoted by reference number 289), the method (denoted by reference number 202) may comprise updating, specifically changing, the blacklist information on the individual mobile device 112 and/or the model group (denoted by reference number 290), particularly in a manner that the individual mobile device 112 and/or a model group is no longer blacklisted. Particularly in case a software update is not released, the method (denoted by reference number 202) may comprise maintaining the blacklist information on the individual mobile device 112 and/or the model group, particularly in a manner that the individual mobile device 112 and/or a model group remains blacklisted (denoted by reference number 286).

Particularly in case the blacklist information on the individual mobile device 112 and/or the model group was updated, the method (denoted by reference number 202) may comprise updating the threshold condition to an updated threshold condition (denoted by reference number 292), particularly an updated threshold condition different from the threshold condition, more particularly an updated threshold condition having a decreased demand for blacklisting the model group and/or individual mobile device 112 again. Exemplarily, a model group may be blacklisted again after only one, two and/or three failure(s) of a mobile device 112 associated with this model group in an on-board suitability check, particularly a failure related to the specific software.

An analytical method, particularly wherein at least one step of the method is computer implemented method, particularly at least one of the steps 302; or 304, for determining at least one parameter of a sample of a bodily fluid (denoted by reference number 294), specifically for detecting at least one analyte in the bodily fluid, is illustrated in an exemplary embodiment in Figure 4. The method comprises the following steps:
- optionally providing at least one optical test element 120 (denoted by reference number 296) having at least one test field 122 configured for performing at least one optically detectable detection reaction in the presence of at least one analyte in a sample of bodily fluid applied to the optical test element 120;
- optionally applying at least one sample of bodily fluid (denoted by reference number 298) to the optical test element 120;
- optionally providing at least one mobile device 112 (denoted by reference number 300) having at least one camera 116;
- running (denoted by reference number 302), on the mobile device 112, the computer-implemented method of operating a mobile device (denoted by reference number 204),
- in case the mobile device 112 is providing clearance information for the analytical measurement in step f, receiving an image of the optical test element 120 by the camera 116 of the mobile device 112, wherein the image comprises at least a part of the test field 122 having the sample of bodily fluid applied thereto.

### List of reference numbers

- 110: analytical system
- 112: mobile device
- 114: cloud server
- 116: camera
- 118: processor
- 120: optical test element
- 122: test field
- 124: cloud-based blacklist database
- 200: computer-implemented method of operating a system
- 202: computer-implemented method of operating a cloud server
- 204: computer-implemented method of operating a mobile device
- 206: providing a cloud-based blacklist database
- 208: sending request by a requesting mobile device
- 209: receiving request from the requesting mobile device
- 210: providing at least one item of blacklist information
- 211: receiving at least one item of blacklist information
- 212: providing at least one item of blacklist information
- 213: receiving at least one item of blacklist information
- 214: providing at least one item of blacklist information
- 215: receiving at least one item of blacklist information
- 216: receiving at least one item of individual check result information
- 218: evaluating the item of individual check result information
- 220: considering a model group of the mobile device for providing model-related blacklist information
- 222: considering the individual mobile device for providing individual blacklist information
- 224: recording at least a part of individual check result information from the mobile device
- 226: comparing an individual absolute number of failures with a predetermined individual absolute failure threshold
- 228: comparing a model absolute number of failures with a predetermined model absolute failure threshold
- 230: comparing an individual relative number of failures with a predetermined individual relative failure threshold
- 232: comparing a model relative number of failures with a predetermined model relative failure threshold
- 234: blacklisting the individual mobile device
- 236: blacklisting the model group
- 237: evaluating the item of blacklist information
- 238: preventing the analytical measurement
- 240: initiating and/or performing at least one on-board suitability check
- 242: evaluating the result of the on-board suitability check
- 244: preventing the analytical measurement
- 246: sending individual check result information
- 248: providing clearance information and/or performing the analytic measurement
- 250: recording at least one image
- 252: determining at least one parameter
- 254: applying a failsafe operation
- 256: sending additional individual check result information
- 258: generating at least one indication that the mobile device is unsuited for the analytical measurement
- 260: generating at least one indication that the mobile device is unsuited for the analytical measurement
- 262: checking the mobile device's hardware
- 264: checking the camera's color resolution of the mobile device
- 266: checking the camera's spatial resolution of the mobile device
- 268: comparing at least one item of information derived from a reference image with at least one predetermined reference criterion
- 270: generating at least one item of information about the mobile device
- 272: transmitting item of information to the cloud server
- 274: selecting the item of information on the mobile device
- 276: initiating the method, particularly by a user
- 278: evaluating the result of the failsafe operation
- 280: preventing and/or aborting the analytical measurement
- 282: displaying the result of the analytical measurement
- 284: determining whether the suitability for performing an analyte measurement is related to a specific software
- 286: maintaining blacklist information in the individual mobile device and/or the model group
- 288: monitoring software updates of the mobile devices
- 289: evaluating if a software update is released
- 290: updating blacklist information in the individual mobile device and/or the model group
- 292: updating the threshold condition
- 294: analytical method for determining at least one parameter of a sample of a bodily fluid
- 296: providing at least one optical test element
- 298: applying at least one sample of bodily fluid
- 300: providing at least one mobile device
- 302: running the computer-implemented method of operating a mobile device
- 304: determining at least one parameter of the sample of a bodily fluid

## Claims

1. A computer-implemented method of operating a cloud server (114) for providing blacklist information, the method comprising:
i. providing a cloud-based blacklist database (124) on the cloud server (114), the blacklist database comprising blacklist information on a plurality of mobile devices (112), each mobile device (112) having at least one camera (116), the blacklist information comprising information on the suitability of at least one of the mobile devices (112)' hardware and the mobile devices (112)' software for performing at least one analytical measurement;
ii. providing, upon request by a requesting mobile device (112) of the plurality of mobile devices (112), at least one item of blacklist information from the blacklist database to the requesting mobile device (112), the blacklist information comprising information on the suitability of at least one of the requesting mobile device's hardware and the requesting mobile device's software for performing the at least one analytical measurement, wherein the request from the requesting mobile device (112) comprises at least one item of information on the mobile device (112) comprising an individual identifier of the mobile device (112) and the model type of the mobile device (112);
iii. receiving at least one item of individual check result information from a sending mobile device (112) of the plurality of mobile devices (112), the item of check result information comprising information on the suitability of at least one of the sending mobile device's hardware and the sending mobile device's software for performing the at least one analytical measurement;
iv. evaluating the item of individual check result information for the necessity of updating the blacklist information on the blacklist database;
wherein the mobile devices (112) of the plurality of mobile devices (112) are grouped into model groups, wherein the blacklist information comprises model-related blacklist information and individual blacklist information, wherein the model-related blacklist information comprises, for the individual model groups, information on the suitability of at least one of the hardware and the software of the mobile devices (112) belonging to the respective model group for performing the at least one analytical measurement, and wherein the individual blacklist information comprises individual information on the suitability of at least one of the hardware and the software of individual mobile devices (112) of the plurality of mobile devices (112) for performing the at least one analytical measurement.

2. The method according to the preceding claim, wherein the method comprises recording at least a part of the individual check result information from sending mobile devices (112) having sent individual check result information to the cloud server (114), wherein the recording comprises updating at least one check result counter with each sending of individual check result information by a sending mobile device (112) of the plurality of mobile devices (112), wherein the check result counter comprises at least one of a model check result counter for a model group of the sending mobile device (112) and an individual check result counter for the individual sending mobile device (112).

3. The method according to the preceding claim, wherein step iv. comprises subjecting the recorded part of the individual check result information for the individual sending mobile devices (112) to at least one threshold condition.

4. A computer-implemented method of operating a mobile device (112) having at least one camera (116), the method comprising:
a. the mobile device (112) requesting, from at least one cloud server (114), at least one item of blacklist information, the blacklist information comprising information on the suitability of at least one of the mobile device's hardware and the mobile device's software for performing at least one analytical measurement based on an optical detection reaction, wherein the request from the requesting mobile device (112) comprises at least one item of information on the mobile device (112) comprising an individual identifier of the mobile device (112) and the model type of the mobile device (112);
b. the mobile device (112) evaluating the item of blacklist information;
c. if the result of the evaluation in step b. indicates that the mobile device (112) is unsuited for the analytical measurement, the mobile device (112) preventing the analytical measurement;
d. if the result of the evaluation in step b. indicates that the mobile device (112) is suited for the analytical measurement, the mobile device (112) initiating at least one on-board suitability check for on-board checking the suitability of the mobile device (112) for performing the analytical measurement;
e. if the result of the on-board suitability check in step d. indicates that the mobile device (112) is unsuited for the analytical measurement, the mobile device (112) preventing the analytical measurement and sending individual check result information to the cloud server (114), the individual check result information comprising update information on the failed on-board suitability check of the mobile device (112);
f. if the result of the onboard suitability check in step d. indicates that the mobile device (112) is suited for the analytical measurement, the mobile device (112) providing clearance information for the analytical measurement;
wherein the mobile devices (112) of the plurality of mobile devices (112) are grouped into model groups, wherein the blacklist information comprises model-related blacklist information and individual blacklist information, wherein the model-related blacklist information comprises, for the individual model groups, information on the suitability of at least one of the hardware and the software of the mobile devices (112) belonging to the respective model group for performing the at least one analytical measurement, and wherein the individual blacklist information comprises individual information on the suitability of at least one of the hardware and the software of individual mobile devices (112) of the plurality of mobile devices (112) for performing the at least one analytical measurement.

5. The computer-implemented method of operating a mobile device (112) according to the preceding claim, wherein step f. further comprises sending individual check result information to the cloud server (114), the individual check result information comprising update information on the passed on-board suitability check of the mobile device (112), the passed on-board suitability check of the mobile device (112) indicating that the mobile device (112) is suited for performing the analytical measurement.

6. The computer-implemented method of operating a mobile device (112) according to any one of the preceding claims referring to a computer-implemented method of operating a mobile device (112), wherein the on-board suitability check in step d. comprises at least one of: checking if the mobile device's hardware complies with one or more predetermined hardware compliance criteria; checking if the camera's color resolution complies with one or more predetermined color resolution criteria; checking if the camera's spatial resolution complies with one or more spatial resolution criteria.

7. The computer-implemented method of operating a mobile device (112) according to any one of the preceding claims referring to a computer-implemented method of operating a mobile device (112), wherein the on-board suitability check in step d. comprises capturing at least one reference image of a reference card and comparing at least one item of information derived from the reference image with at least one predetermined reference criterion.

8. An analytical method for determining at least one parameter of a sample of a bodily fluid, the method comprising:
- running the computer-implemented method of operating a mobile device (112) according to any one of the preceding claims referring to a computer-implemented method of operating a mobile device (112) on the mobile device (112), wherein the mobile device (112) has at least one camera (116),
- in case the mobile device (112) is providing clearance information for the analytical measurement in step f, receiving an image of the optical test element (120) by the camera (116) of the mobile device (112), wherein the optical test element (120) has at least one test field (122) configured for performing at least one optically detectable detection reaction in the presence of at least one analyte in a sample of bodily fluid applied to the optical test element (120), wherein the image comprises at least a part of the test field (122) having the sample of bodily fluid applied thereto.

9. A computer-implemented method of operating a system comprising at least one cloud server (114) and a plurality of mobile devices (112), each mobile device (112) having at least one camera (116), the method comprising:
- operating the cloud server (114) by using the method according to any one of the preceding claims referring to a computer-implemented method of operating a cloud server (114) for providing blacklist information; and
- operating at least one of the mobile devices (112) of the plurality of mobile devices (112) by using the method according to any one of the preceding claims referring to a computer-implemented method of operating a mobile device (112).

10. A cloud server (114) for providing blacklist information, the cloud server (114) being configured for performing the computer-implemented method of operating a cloud server (114) according to any one of the preceding claims referring to a computer-implemented method of operating a cloud server (114) for providing blacklist information.

11. A computer program comprising instructions which, when the program is executed by the cloud server (114) according to the preceding claim, cause the cloud server (114) to perform the computer-implemented method of operating a cloud server (114) for providing blacklist information.

12. A mobile device (112) having at least one camera (116), the mobile device (112) being configured for performing the computer-implemented method of operating a mobile device (112) according to any one of the preceding claims referring to a computer-implemented method of operating a mobile device (112) having at least one camera (116).

13. A computer program comprising instructions which, when the program is executed by the mobile device (112) according to the preceding claim, cause the mobile device (112) to perform the computer-implemented method of operating a mobile device (112) according to any one of the preceding claims referring to a computer-implemented method of operating a mobile device (112).

14. An analytical system (110) comprising at least one mobile device (112) having at least one camera (116) and at least one cloud server (114) according to any one of the preceding claims referring to a mobile device (112) and a cloud server, respectively; and optionally comprising at least one optical test element (120).

## Patentansprüche

1. Computerimplementiertes Verfahren zum Betreiben eines Cloud-Servers (114) zum Bereitstellen von Blacklist-Informationen, wobei das Verfahren Folgendes umfasst:
i. Bereitstellen einer cloudbasierten Blacklist-Datenbank (124) auf dem Cloud-Server (114), wobei die Blacklist-Datenbank Blacklist-Informationen über eine Vielzahl von mobilen Vorrichtungen (112) umfasst, wobei jede mobile Vorrichtung (112) mindestens eine Kamera (116) aufweist, wobei die Blacklist-Informationen Informationen über die Eignung mindestens einer von Hardware der mobilen Vorrichtungen (112) und Software der mobilen Vorrichtungen (112) zum Durchführen mindestens einer analytischen Messung umfassen;
ii. Bereitstellen mindestens eines Blacklist-Informationselements aus der Blacklist-Datenbank auf Anforderung durch eine anfordernde mobile Vorrichtung (112) der Vielzahl von mobilen Vorrichtungen (112) für die anfordernde mobile Vorrichtung (112), wobei die Blacklist-Informationen Informationen über die Eignung mindestens einer von Hardware der anfordernden mobilen Vorrichtung und Software der anfordernden mobilen Vorrichtung zum Durchführen der mindestens einen analytischen Messung umfassen, wobei die Anforderung von der anfordernden mobilen Vorrichtung (112) mindestens ein Informationselement über die mobile Vorrichtung (112) umfasst, das eine individuelle Kennung der mobilen Vorrichtung (112) und den Modelltyp der mobilen Vorrichtung (112) umfasst;
iii. Empfangen mindestens eines individuellen Prüfergebnis-Informationselements von einer sendenden mobilen Vorrichtung (112) der Vielzahl von mobilen Vorrichtungen (112), wobei das Prüfergebnis-Informationselement Informationen über die Eignung mindestes einer von Hardware der sendenden mobilen Vorrichtung und Software der sendenden mobilen Vorrichtung zum Durchführen der mindestens einen analytischen Messung umfasst;
iv. Bewerten des individuellen Prüfergebnis-Informationselements auf die Notwendigkeit des Aktualisierens der Blacklist-Informationen in der Blacklist-Datenbank;
wobei die mobilen Vorrichtungen (112) der Vielzahl von mobilen Vorrichtungen (112) in Modellgruppen gruppiert sind, wobei die Blacklist-Informationen modellbezogene Blacklist-Informationen und individuelle Blacklist-Informationen umfassen, wobei die modellbezogenen Blacklist-Informationen für die individuellen Modellgruppen Informationen über die Eignung mindestens einer von Hardware und Software der mobilen Vorrichtungen (112), die zu der jeweiligen Modellgruppe gehören, zum Durchführen der mindestens einen analytischen Messung umfassen und wobei die individuellen Blacklist-Informationen individuelle Informationen über die Eignung mindestens einer von Hardware und Software individueller mobiler Vorrichtungen (112) der Vielzahl von mobilen Vorrichtungen (112) zum Durchführen der mindestens einen analytischen Messung umfassen.

2. Verfahren nach dem vorhergehenden Anspruch, wobei das Verfahren das Aufzeichnen mindestens eines Teils der individuellen Prüfergebnis-Informationen von sendenden mobilen Vorrichtungen (112), die individuelle Prüfergebnis-Informationen an den Cloud-Server (114) gesendet haben, umfasst, wobei das Aufzeichnen das Aktualisieren mindestens eines Prüfergebnis-Zählers mit jedem Senden individueller Prüfergebnis-Informationen durch eine sendende mobile Vorrichtung (112) der Vielzahl von mobilen Vorrichtungen (112) umfasst, wobei der Prüfergebnis-Zähler mindestens eines von einem Modellprüfergebnis-Zähler für eine Modellgruppe der sendenden mobilen Vorrichtung (112) und einem individuellen Prüfergebnis-Zähler für die individuelle sendende mobile Vorrichtung (112) umfasst.

3. Verfahren nach dem vorhergehenden Anspruch, wobei Schritt iv. das Unterwerfen des aufgezeichneten Teils der individuellen Prüfergebnis-Informationen für die individuellen sendenden mobilen Vorrichtungen (112) mindestens einer Schwellenbedingung umfasst.

4. Computerimplementiertes Verfahren zum Betreiben einer mobilen Vorrichtung (112), die mindestens eine Kamera (116) aufweist, wobei das Verfahren Folgendes umfasst:
a. Anfordern mindestens eines Blacklist-Informationselements von mindestens einem Cloud-Server (114) durch die mobile Vorrichtung (112), wobei die Blacklist-Informationen Informationen über die Eignung mindestens einer von Hardware der mobilen Vorrichtung und Software der mobilen Vorrichtung zum Durchführen mindestens einer analytischen Messung basierend auf einer optischen Detektionsreaktion umfassen, wobei die Anforderung von der anfordernden mobilen Vorrichtung (112) mindestens ein Informationselement über die mobile Vorrichtung (112) umfasst, das eine individuelle Kennung der mobilen Vorrichtung (112) und den Modelltyp der mobilen Vorrichtung (112) umfasst;
b. Bewerten des Blacklist-Informationselements durch die mobile Vorrichtung (112);
c. wenn das Ergebnis der Bewertung in Schritt b. angibt, dass die mobile Vorrichtung (112) für die analytische Messung ungeeignet ist, Verhindern der analytischen Messung durch die mobile Vorrichtung (112);
d. wenn das Ergebnis der Bewertung in Schritt b. angibt, dass die mobile Vorrichtung (112) für die analytische Messung geeignet ist, Initiieren mindestens einer On-Board-Eignungsprüfung durch die mobile Vorrichtung (112) zum On-Board-Prüfen der Eignung der mobilen Vorrichtung (112) zum Durchführen der analytischen Messung;
e. wenn das Ergebnis der On-Board-Eignungsprüfung in Schritt d. angibt, dass die mobile Vorrichtung (112) für die analytische Messung ungeeignet ist, Verhindern der analytischen Messung durch die mobile Vorrichtung (112) und Senden individueller Prüfergebnis-Informationen an den Cloud-Server (114), wobei die individuellen Prüfergebnis-Informationen Aktualisierungsinformationen über die fehlgeschlagene On-Board-Eignungsprüfung der mobilen Vorrichtung (112) umfassen;
f. wenn das Ergebnis der On-Board-Eignungsprüfung in Schritt d. angibt, dass die mobile Vorrichtung (112) für die analytische Messung geeignet ist, Bereitstellen von Freigabeinformationen für die analytische Messung durch die mobile Vorrichtung (112);
wobei die mobilen Vorrichtungen (112) der Vielzahl von mobilen Vorrichtungen (112) in Modellgruppen gruppiert sind, wobei die Blacklist-Informationen modellbezogene Blacklist-Informationen und individuelle Blacklist-Informationen umfassen, wobei die modellbezogenen Blacklist-Informationen für die individuellen Modellgruppen Informationen über die Eignung mindestens einer von Hardware und Software der mobilen Vorrichtungen (112), die zu der jeweiligen Modellgruppe gehören, zum Durchführen der mindestens einen analytischen Messung umfassen und wobei die individuellen Blacklist-Informationen individuelle Informationen über die Eignung mindestens einer von Hardware und Software individueller mobiler Vorrichtungen (112) der Vielzahl von mobilen Vorrichtungen (112) zum Durchführen der mindestens einen analytischen Messung umfassen.

5. Computerimplementiertes Verfahren zum Betreiben einer mobilen Vorrichtung (112) nach dem vorhergehenden Anspruch, wobei Schritt f. ferner das Senden individueller Prüfergebnis-Informationen an den Cloud-Server (114) umfasst, wobei die individuellen Prüfergebnis-Informationen Aktualisierungsinformationen über die bestandene On-Board-Eignungsprüfung der mobilen Vorrichtung (112) umfassen, wobei die bestandene On-Board-Eignungsprüfung der mobilen Vorrichtung (112) angibt, dass die mobile Vorrichtung (112) zum Durchführen der analytischen Messung geeignet ist.

6. Computerimplementiertes Verfahren zum Betreiben einer mobilen Vorrichtung (112) nach einem der vorhergehenden Ansprüche, die sich auf ein computerimplementiertes Verfahren zum Betreiben einer mobilen Vorrichtung (112) beziehen, wobei die On-Board-Eignungsprüfung in Schritt d. mindestens eines der Folgenden umfasst: Prüfen, ob die Hardware der mobilen Vorrichtung ein oder mehrere vorbestimmte Hardware-Konformitätskriterien erfüllt; Prüfen, ob die Farbauflösung der Kamera ein oder mehrere vorbestimmte Farbauflösungskriterien erfüllt; Prüfen, ob die räumliche Auflösung der Kamera ein oder mehrere Kriterien für räumliche Auflösung erfüllt.

7. Computerimplementiertes Verfahren zum Betreiben einer mobilen Vorrichtung (112) nach einem der vorhergehenden Ansprüche, die sich auf ein computerimplementiertes Verfahren zum Betreiben einer mobilen Vorrichtung (112) beziehen, wobei die On-Board-Eignungsprüfung in Schritt d. das Erfassen mindestens eines Referenzbilds einer Referenzkarte und das Vergleichen mindestens eines Informationselements, das von dem Referenzbild abgeleitet ist, mit mindestens einem vorbestimmten Referenzkriterium umfasst.

8. Analytisches Verfahren zum Bestimmen mindestens eines Parameters einer Probe einer Körperflüssigkeit, wobei das Verfahren Folgendes umfasst:
- Ausführen des computerimplementierten Verfahrens zum Betreiben einer mobilen Vorrichtung (112) nach einem der vorhergehenden Ansprüche, die sich auf ein computerimplementiertes Verfahren zum Betreiben einer mobilen Vorrichtung (112) beziehen, auf der mobilen Vorrichtung (112), wobei die mobile Vorrichtung (112) mindestens eine Kamera (116) aufweist,
- falls die mobile Vorrichtung (112) Freigabeinformationen für die analytische Messung in Schritt f. bereitstellt, Empfangen eines Bilds des optischen Testelements (120) durch die Kamera (116) der mobilen Vorrichtung (112), wobei das optische Testelement (120) mindestens ein Testfeld (122) aufweist, das zum Durchführen mindestens einer optisch detektierbaren Detektionsreaktion in Gegenwart mindestens eines Analyten in einer Probe der Körperflüssigkeit, die auf das optische Testelement (120) aufgebracht wird, konfiguriert ist, wobei das Bild mindestens einen Teil des Testfelds (122) umfasst, auf das die Probe der Körperflüssigkeit aufgebracht wird.

9. Computerimplementiertes Verfahren zum Betreiben eines Systems, das mindestens einen Cloud-Server (114) und eine Vielzahl von mobilen Vorrichtungen (112) umfasst, wobei jede mobile Vorrichtung (112) mindestens eine Kamera (116) aufweist, wobei das Verfahren Folgendes umfasst:
- Betreiben des Cloud-Servers (114) unter Verwendung des Verfahrens nach einem der vorhergehenden Ansprüche, die sich auf ein computerimplementiertes Verfahren zum Betreiben eines Cloud-Servers (114) zum Bereitstellen von Blacklist-Informationen beziehen; und
- Betreiben mindestens eine der mobilen Vorrichtungen (112) der Vielzahl von mobilen Vorrichtungen (112) unter Verwendung des Verfahrens nach einem der vorhergehenden Ansprüche, die sich auf ein computerimplementiertes Verfahren zum Betreiben einer mobilen Vorrichtung (112) beziehen.

10. Cloud-Server (114) zum Bereitstellen von Blacklist-Informationen, wobei der Cloud-Server (114) zum Durchführen des computerimplementierten Verfahrens zum Betreiben eines Cloud-Servers (114) nach einem der vorhergehenden Ansprüche konfiguriert ist, die sich auf ein computerimplementiertes Verfahren zum Betreiben eines Cloud-Servers (114) zum Bereitstellen von Blacklist-Informationen beziehen.

11. Computerprogramm, das Anweisungen umfasst, die, wenn das Programm durch den Cloud-Server (114) nach dem vorhergehenden Anspruch ausgeführt wird, den Cloud-Server (114) veranlassen, das computerimplementierte Verfahren zum Betreiben eines Cloud-Servers (114) zum Bereitstellen von Blacklist-Informationen durchzuführen.

12. Mobile Vorrichtung (112), die mindestens eine Kamera (116) aufweist, wobei die mobile Vorrichtung (112) zum Durchführen des computerimplementierten Verfahrens zum Betreiben einer mobilen Vorrichtung (112) nach einem der vorhergehenden Ansprüche konfiguriert ist, die sich auf ein computerimplementiertes Verfahren zum Betreiben einer mobilen Vorrichtung (112), die mindestens eine Kamera (116) aufweist, beziehen.

13. Computerprogramm, das Anweisungen umfasst, die, wenn das Programm durch die mobile Vorrichtung (112) nach dem vorhergehenden Anspruch ausgeführt wird, die mobile Vorrichtung (112) veranlassen, das computerimplementierte Verfahren zum Betreiben einer mobilen Vorrichtung (112) nach einem der vorhergehenden Ansprüche durchzuführen, die sich auf ein computerimplementiertes Verfahren zum Betreiben einer mobilen Vorrichtung (112) beziehen.

14. Analytisches System (110), das mindestens eine mobile Vorrichtung (112), die mindestens eine Kamera (116) aufweist, und mindestens einen Cloud-Server (114) nach einem der vorhergehenden Ansprüche umfasst, die sich auf eine mobile Vorrichtung (112) bzw. einen Cloud-Server beziehen; und optional mindestens ein optisches Testelement (120) umfasst.

## Revendications

1. Procédé mis en œuvre par ordinateur de fonctionnement d'un serveur cloud (114) pour fournir des informations de liste noire, le procédé comprenant :
i. la fourniture d'une base de données de liste noire basée sur le cloud (124) sur le serveur cloud (114), la base de données de liste noire comprenant des informations de liste noire sur une pluralité de dispositifs mobiles (112), chaque dispositif mobile (112) ayant au moins une caméra (116), les informations de liste noire comprenant des informations sur la pertinence d'au moins un parmi le matériel des dispositifs mobiles (112) et le logiciel des dispositifs mobiles (112) pour effectuer au moins une mesure analytique ;
ii. la fourniture, lors de la demande par un dispositif mobile (112) demandeur de la pluralité de dispositifs mobiles (112), d'au moins un élément d'informations de liste noire provenant de la base de données de liste noire au dispositif mobile (112) demandeur, les informations de liste noire comprenant des informations sur la pertinence d'au moins un parmi le matériel du dispositif mobile demandeur et le logiciel du dispositif mobile demandeur pour effectuer l'au moins une mesure analytique, dans lequel la demande provenant du dispositif mobile (112) demandeur comprend au moins un élément d'informations sur le dispositif mobile (112) comprenant un identifiant individuel du dispositif mobile (112) et le type de modèle du dispositif mobile (112) ;
iii. la réception d'au moins un élément d'informations de résultats de vérification individuelle provenant d'un dispositif mobile (112) émetteur de la pluralité de dispositifs mobiles (112), l'élément d'informations de résultats de vérification comprenant des informations sur la pertinence d'au moins un parmi le matériel du dispositif mobile émetteur et le logiciel du dispositif mobile émetteur pour effectuer l'au moins une mesure analytique ;
iv. l'évaluation de l'élément d'informations de résultats de vérification individuelle quant à la nécessité de mettre à jour les informations de liste noire sur la base de données de liste noire ;
dans lequel les dispositifs mobiles (112) de la pluralité de dispositifs mobiles (112) sont regroupés en groupes de modèles, dans lequel les informations de liste noire comprennent des informations de liste noire liées au modèle et des informations de liste noire individuelles, dans lequel les informations de liste noire liées au modèle comprennent, pour les groupes de modèles individuels, des informations sur la pertinence d'au moins un parmi le matériel et le logiciel des dispositifs mobiles (112) appartenant au groupe de modèles respectif pour effectuer l'au moins une mesure analytique, et dans lequel les informations de liste noire individuelles comprennent des informations individuelles sur la pertinence d'au moins un parmi le matériel et le logiciel des dispositifs mobiles (112) individuels de la pluralité de dispositifs mobiles (112) pour effectuer l'au moins une mesure analytique.

2. Procédé selon la revendication précédente, dans lequel le procédé comprend l'enregistrement d'au moins une partie des informations de résultats de vérification individuelle provenant des dispositifs mobiles (112) émetteurs ayant envoyé des informations de résultats de vérification individuelle au serveur cloud (114), dans lequel l'enregistrement comprend la mise à jour d'au moins un compteur de résultats de vérification avec chaque envoi d'informations de résultats de vérification individuelle par un dispositif mobile (112) émetteur de la pluralité de dispositifs mobiles (112), dans lequel le compteur de résultats de vérification comprend au moins un parmi un compteur de résultats de vérification de modèle pour un groupe de modèles du dispositif mobile (112) émetteur et un compteur de résultats de vérification individuelle pour le dispositif mobile (112) émetteur individuel.

3. Procédé selon la revendication précédente, dans lequel l'étape iv. comprend la soumission de la partie enregistrée des informations de résultats de vérification individuelle pour les dispositifs mobiles (112) émetteurs individuels à au moins une condition de seuil.

4. Procédé mis en œuvre par ordinateur de fonctionnement d'un dispositif mobile (112) ayant au moins une caméra (116), le procédé comprenant :
a. le dispositif mobile (112) demandant, provenant d'au moins un serveur cloud (114), au moins un élément d'informations de liste noire, les informations de liste noire comprenant des informations sur la pertinence d'au moins un parmi le matériel du dispositif mobile et le logiciel du dispositif mobile pour effectuer au moins une mesure analytique en se basant sur une réaction de détection optique, dans lequel la demande du dispositif mobile (112) demandeur comprend au moins un élément d'informations sur le dispositif mobile (112) comprenant un identifiant individuel du dispositif mobile (112) et le type de modèle du dispositif mobile (112) ;
b. le dispositif mobile (112) évaluant l'élément d'informations de liste noire ;
c. si le résultat de l'évaluation de l'étape b. indique que le dispositif mobile (112) n'est pas pertinent pour la mesure analytique, le dispositif mobile (112) empêchant la mesure analytique ;
d. si le résultat de l'évaluation de l'étape b. indique que le dispositif mobile (112) est pertinent pour la mesure analytique, le dispositif mobile (112) initiant au moins une vérification de pertinence embarquée pour vérifier de manière embarquée la pertinence du dispositif mobile (112) pour effectuer la mesure analytique ;
e. si le résultat de vérification de pertinence embarquée de l'étape d. indique que le dispositif mobile (112) n'est pas pertinent pour la mesure analytique, le dispositif mobile (112) empêchant la mesure analytique et envoyant des informations de résultats de vérification individuelle au serveur cloud (114), les informations de résultats de vérification individuelle comprenant des informations de mise à jour sur la vérification de pertinence embarquée ayant échoué du dispositif mobile (112) ;
f. si le résultat de vérification de pertinence embarquée de l'étape d. indique que le dispositif mobile (112) est pertinent pour la mesure analytique, le dispositif mobile (112) fournissant des informations d'autorisation pour la mesure analytique ;
dans lequel les dispositifs mobiles (112) de la pluralité de dispositifs mobiles (112) sont regroupés en groupes de modèles, dans lequel les informations de liste noire comprennent des informations de liste noire liées au modèle et des informations de liste noire individuelles, dans lequel les informations de liste noire liées au modèle comprennent, pour les groupes de modèles individuels, des informations sur la pertinence d'au moins un parmi le matériel et le logiciel des dispositifs mobiles (112) appartenant au groupe de modèles respectif pour effectuer l'au moins une mesure analytique, et dans lequel les informations de liste noire individuelles comprennent des informations individuelles sur la pertinence d'au moins un parmi le matériel et le logiciel des dispositifs mobiles (112) individuels de la pluralité de dispositifs mobiles (112) pour effectuer l'au moins une mesure analytique.

5. Procédé mis en œuvre par ordinateur de fonctionnement d'un dispositif mobile (112) selon la revendication précédente, dans lequel l'étape f. comprend en outre l'envoi d'informations de résultats de vérification individuelle au serveur cloud (114), les informations de résultats de vérification individuelle comprenant des informations de mise à jour sur la vérification de pertinence embarquée réussie du dispositif mobile (112), la vérification de pertinence embarquée réussie du dispositif mobile (112) indiquant que le dispositif mobile (112) est pertinent pour effectuer la mesure analytique.

6. Procédé mis en œuvre par ordinateur de fonctionnement d'un dispositif mobile (112) selon l'une quelconque des revendications précédentes se référant à un procédé mis en œuvre par ordinateur de fonctionnement d'un dispositif mobile (112), dans lequel la vérification de pertinence embarquée de l'étape d. comprend au moins un parmi : la vérification si le matériel du dispositif mobile est conforme à un ou plusieurs critères de conformité de matériel prédéterminés ; la vérification si la résolution colorimétrique de la caméra est conforme à un ou plusieurs critères de résolution colorimétrique prédéterminés ; la vérification si la résolution spatiale de la caméra est conforme à un ou plusieurs critères de résolution spatiale.

7. Procédé mis en œuvre par ordinateur de fonctionnement d'un dispositif mobile (112) selon l'une quelconque des revendications précédentes se référant à un procédé mis en œuvre par ordinateur de fonctionnement d'un dispositif mobile (112), dans lequel la vérification de pertinence embarquée de l'étape d. comprend la capture d'au moins une image de référence d'une carte de référence et la comparaison d'au moins un élément d'informations dérivé de l'image de référence à au moins un critère de référence prédéterminé.

8. Procédé analytique pour déterminer au moins un paramètre d'un échantillon d'un fluide corporel, le procédé comprenant :
- l'exécution du procédé mis en œuvre par ordinateur de fonctionnement d'un dispositif mobile (112) selon l'une quelconque des revendications précédentes se référant à un procédé mis en œuvre par ordinateur de fonctionnement d'un dispositif mobile (112) sur le dispositif mobile (112), dans lequel le dispositif mobile (112) a au moins une caméra (116),
- dans le cas où le dispositif mobile (112) fournit des informations d'autorisation pour la mesure analytique de l'étape f, la réception d'une image de l'élément de test optique (120) par la caméra (116) du dispositif mobile (112), dans lequel l'élément de test optique (120) a au moins un champ de test (122) configuré pour effectuer au moins une réaction de détection détectable de manière optique en présence d'au moins un analyte dans un échantillon de fluide corporel appliqué sur l'élément de test optique (120), dans lequel l'image comprend au moins une partie du champ de test (122) ayant l'échantillon de fluide corporel appliqué sur celui-ci.

9. Procédé mis en œuvre par ordinateur de fonctionnement d'un système comprenant au moins un serveur cloud (114) et une pluralité de dispositifs mobiles (112), chaque dispositif mobile (112) ayant au moins une caméra (116), le procédé comprenant :
- le fonctionnement du serveur cloud (114) en utilisant le procédé selon l'une quelconque des revendications précédentes se référant à un procédé mis en œuvre par ordinateur de fonctionnement d'un serveur cloud (114) pour fournir des informations de liste noire ; et
- le fonctionnement d'au moins un des dispositifs mobiles (112) de la pluralité de dispositifs mobiles (112) en utilisant le procédé selon l'une quelconque des revendications précédentes se référant à un procédé mis en œuvre par ordinateur de fonctionnement d'un dispositif mobile (112).

10. Serveur cloud (114) pour fournir des informations de liste noire, le serveur cloud (114) étant configuré pour effectuer le procédé mis en œuvre par ordinateur de fonctionnement d'un serveur cloud (114) selon l'une quelconque des revendications précédentes se référant à un procédé mis en œuvre par ordinateur de fonctionnement d'un serveur cloud (114) pour fournir des informations de liste noire.

11. Programme informatique comprenant des instructions qui, lorsque le programme est exécuté par le serveur cloud (114) selon la revendication précédente, amènent le serveur cloud (114) à effectuer le procédé mis en œuvre par ordinateur de fonctionnement d'un serveur cloud (114) pour fournir des informations de liste noire.

12. Dispositif mobile (112) ayant au moins une caméra (116), le dispositif mobile (112) étant configuré pour effectuer le procédé mis en œuvre par ordinateur de fonctionnement d'un dispositif mobile (112) selon l'une quelconque des revendications précédentes se référant à un procédé mis en œuvre par ordinateur de fonctionnement d'un dispositif mobile (112) ayant au moins une caméra (116).

13. Programme informatique comprenant des instructions qui, lorsque le programme est exécuté par le dispositif mobile (112) selon la revendication précédente, amènent le dispositif mobile (112) à effectuer le procédé mis en œuvre par ordinateur de fonctionnement d'un dispositif mobile (112) selon l'une quelconque des revendications précédentes se référant à un procédé mis en œuvre par ordinateur de fonctionnement d'un dispositif mobile (112).

14. Système analytique (110) comprenant au moins un dispositif mobile (112) ayant au moins une caméra (116) et au moins un serveur cloud (114) selon l'une quelconque des revendications précédentes se référant à un dispositif mobile (112) et un serveur cloud, respectivement ; et éventuellement comprenant au moins un élément de test optique (120).
